(19) Europäisches Patentamt
European Patent Office
Office européen des brevets

(11) Veröffentlichungsnummer : **0 526 395 A1**

# (12) EUROPÄISCHE PATENTANMELDUNG

(21) Anmeldenummer : **92810536.0**

(22) Anmeldetag : **15.07.92**

(51) Int. Cl.[5] : **C07D 213/38,** C07D 213/74, C07D 277/42, C07D 401/12, C07D 405/12, C07D 417/12, A61K 31/425, A61K 31/44, // (C07D417/12, 277:00, 239:00), (C07D405/12, 307:00, 213:00), (C07D401/12, 233:00, 213:00)

(30) Priorität : **22.07.91 CH 2193/91**

(43) Veröffentlichungstag der Anmeldung :
**03.02.93 Patentblatt 93/05**

(84) Benannte Vertragsstaaten :
**AT BE CH DE DK ES FR GB GR IT LI LU NL PT SE**

(71) Anmelder : **Zyma SA**
**Route de l'Etraz**
**CH-1260 Nyon (CH)**

(72) Erfinder : **Alisch, Rudi A., Dr.**
**Knobelsdorffstrasse 43**
**W-1000 Berlin 19 (DE)**
Erfinder : **Schulze, Frank R., Dr.**
**Kirchstrasse 78**
**W-1000 Berlin 48 (DE)**
Erfinder : **Buschauer, Armin, Dr.**
**Bachestrasse 5**
**W-1000 Berlin 41 (DE)**
Erfinder : **Schunack, Walter, Prof. Dr.**
**Spanische Allee 95**
**W-1000 Berlin 38 (DE)**

(74) Vertreter : **Schluep, Hans-Peter et al**
**c/o CIBA GEIGY AG Patentabteilung Postfach**
**CH-4002 Basel (CH)**

(54) **Arylalkylaminderivate, Verfahren zu ihrer Herstellung und diese Verbindungen enthaltende Arzneimittel.**

(57) Es werden Arylalkylaminderivate der allgemeinen Formel

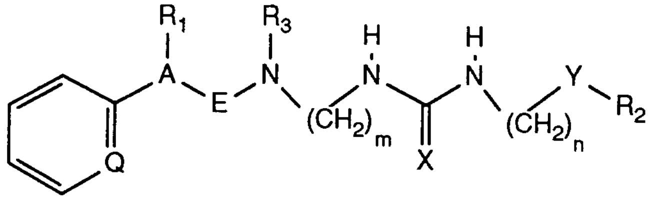

beschrieben, die aufgrund ihrer antagonistischen Wirkung an Histamin-$H_1$ - und $H_2$-Rezeptoren zur Prophylaxe und Therapie von Krankheitszuständen, an denen Histamin beteiligt ist, eingesetzt werden können. Sie werden in an sich bekannter Weise hergestellt.

EP 0 526 395 A1

Jouve, 18, rue Saint-Denis, 75001 PARIS

Gegenstand der Erfindung sind Arylalkylaminderivate, die aufgrund ihrer antagonistischen Wirkung an Histamin-$H_1$- und -$H_2$-Rezeptoren zur Prophylaxe und Therapie von Krankheitszuständen, an denen Histamin beteiligt ist, eingesetzt werden können.

Durch eine Vielzahl von Wirkstoffen, die in Anästhesie und Chirurgie verwendet werden, aber auch durch chirurgische Eingriffe per se, kann es zu einer massiven Freisetzung von Histamin aus Mastzellen kommen. Die Histaminliberation und die nachfolgende Aktivierung von Histamin-$H_1$- und -$H_2$-Rezeptoren können zum klinischen Erscheinungsbild der anaphylaktischen oder anaphylaktoiden Reaktion in verschiedenen Schweregraden führen, von lokaler Erythembildung bis zur systemischen Urtikaria mit schwerwiegendem Blutdruckabfall, Herzarrhythmien und möglicherweise lebensbedrohlichen Bronchospasmen.

Die Stimulation der Histamin-$H_1$-Rezeptoren stellt dabei unter anderem eine Ursache dar für die Kontraktion glatter Muskulatur, beispielsweise der Bronchien, sowie für komplexe kardiovaskuläre Effekte, zum Beispiel Kontraktion oder Dilatation von Gefässen, Erhöhung der Permeabilität des Venolenendothels mit Exsudation und Hämokonzentration, Verlängerung der atrioventrikulären Reizüberleitung bis hin zum AV-Block. Die Aktivierung der $H_2$-Rezeptoren hat unter anderem eine Erhöhung der Herzfrequenz, eine Begünstigung oder Auslösung tachykarder Herzrhythmusstörungen und eine verstärkte Vasodilatation zur Folge. Zudem wird über $H_2$-Rezeptoren der Parietalzellen der Magenschleimhaut die Säuresekretion gesteigert mit der Gefahr pulmonaler Schäden durch Säureaspiration und der Bildung von Stressulcera.

Eine signifikante Reduktion dieser Reaktionen kann durch die kombinierte Verabreichung eines Histamin-$H_1$-Rezeptorantagonisten mit einem $H_2$-Rezeptorantagonisten vor einem chirurgischen Eingriff erreicht werden (Lorenz, W.; Doenicke, A. (1985), "$H_1$- and $H_2$-blockade: A prophylactic principle in anaesthesia and surgery against histamine-release responses of any degree of severity", N. Engl. Reg. Allergy Proc. 6, 37-57. Tryba, M.; Zevounou, F.; Zenz, M. (1986), "Prevention of histamine-induced cardiovascular reactions during the induction of anaesthesia following premedication with $H_1$ - + $H_2$-antagonists i.m.", Br.J. Anaesth. 58, 478-482).

Histamin ist aufgrund seiner oben genannten Wirkungen an $H_1$- und $H_2$-Rezeptoren auch an verschiedenen Erkrankungen beteiligt, wie z.B. entzündlichen Hauterkrankungen; Pruritus (= Jucken) unterschiedlichen Ursprungs, z.B. Pruritus nach Einfluss von Sonnenstrahlung, atopischer Dermatitis und ganz allgemein dermatologischen Krankheiten allergischer Natur, Urtikaria, Allergien, allergischem Asthma; Rhinitis und ganz allgemein Krankheiten der Atmungsorgane, die allergischer Natur sind; Konjunktivitis (= Bindehautentzündung) und ganz allgemein Augenkrankheiten allergischer Natur; und Mastocytose.

Der Erfindung liegt deshalb die Aufgabe zugrunde, Verbindungen bereitzustellen, welche die Wirkungen des Histamins sowohl an den $H_1$- als auch an den $H_2$-Rezeptoren zu hemmen, und die daher z.B. zur Therapie der oben erwähnten, durch Histamin ausgelösten Erkrankungen geeignet sind.

Gegenstand der Erfindung sind Arylalkylaminderivate der allgemeinen Formel I,

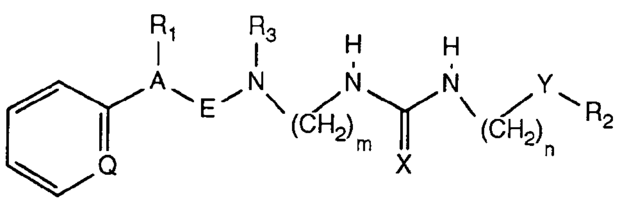

(I)

in der $R_1$ für eine substituierte oder unsubstituierte Aryl-, Heteroaryl-, Aryl-$C_1$-$C_3$-alkyl-oder Heteroaryl-$C_1$-$C_3$-alkyl-gruppe, ein Wasserstoffatom oder eine $C_1$-$C_3$-Alkylgruppe steht, A ein Stickstoffatom oder eine CH-Gruppe bedeutet, E für die Gruppierung $-(CH_2)_p-$, $-O-(CH_2)_p-$, $-S-(CH_2)_p-$ oder

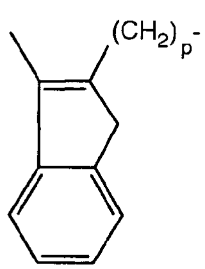

steht, wobei p den Wert 2, 3 oder 4 haben kann, Q für ein Stickstoffatom oder eine CH-Gruppe steht, $R_2$ eine gegebenenfalls basisch substituierte Aryl-, Heteroaryl-, Aryl-$C_1$-$C_3$-alkyl- oder Heteroaryl-$C_1$-$C_3$-alkyl-gruppe,

$R_3$ ein Wasserstoffatom oder eine $C_1$-$C_3$-Alkylgruppe, bevorzugt eine Methylgruppe, X ein Sauerstoffatom, ein Schwefelatom, die Gruppierung N-CN oder $CH$-$NO_2$ bedeutet, m den Wert 2, 3, 4, 5, 6, 7 oder 8 und n den Wert 1, 2, 3 oder 4 haben kann und Y ein Schwefelatom, ein Sauerstoffatom oder eine Methylengruppe bedeutet, sowie pharmazeutisch anwendbare Salze davon, ferner Verfahren zur Herstellung dieser Verbindungen, pharmazeutische Präparate, die diese Verbindungen enthalten, die Verwendung dieser Verbindungen zur therapeutischen Behandlung des menschlichen oder tierischen Körpers oder zur Herstellung pharmazeutischer Präparate.

$C_1$-$C_3$-Alkyl ist z.B. Methyl. Halogen ist z.B. Fluor, Chlor, Brom oder Iod, bevorzugt Fluor oder Chlor. Phenyl-$C_1$-$C_3$-alkyl ist z.B. Benzyl.

Vorzugsweise bedeutet der Rest $R_2$ Piperidino-$C_1$-$C_3$-alkyl-phenyl, Guanidino-thiazolyl-$C_1$-$C_3$-alkyl, $C_1$-$C_3$-Alkyl-imidazolyl-$C_1$-$C_3$-alkyl oder (N,N-Di-$C_1$-$C_3$-alkylamino-$C_1$-$C_3$-alkyl)-furanyl-$C_1$-$C_3$-alkyl.

Die erfindungsgemässen Verbindungen der Formel I zeichnen sich durch eine neuartige, bisher nicht beschriebene pharmakologische Gesamtaktivität aus. Die erfindungsgemässe neue Strukturklasse zeigt sowohl eine Histamin-$H_1$- als auch eine -$H_2$-antagonistische Wirkkomponente. Dies zeigen die folgenden pharmakologischen Ergebnisse. Zur Differenzierung der beiden Wirkqualitäten eignen sich z.B. in vitro Untersuchungen am isolierten spontan schlagenden rechten Atrium ($H_2$) und am isolierten Ileum des Meerschweinchens ($H_1$) (Black, J.W.; Duncan, W.A.M.; Durant, G.J., Ganellin, C.R.; Parsons, M.E. (1972), "Definition and Antagonism of Histamine $H_2$-Receptors". Nature 236, 385-390). Die Aufnahme der Konzentrations-Wirkungskurven zur Ermittlung der pharmakologischen Parameter (- log $K_B$) erfolgte in kumulativer Technik nach van Rossum, J.M. (1963), "Cumulative Dose- Response- Curves. II. Technique for the Making of Dose-Response Curves in Isolated Organs and the Evaluation of Drug Paramters", Arch. Int. Pharmacodyn. Ther. 143, 299-307.

Pharmakologische Daten
(ermittelt am isolierten Ileum bzw. Atrium des Meerschweinchens)

| Verbindungen | $H_1$-Antagonismus Ileum -log $K_B$ | $H_2$-Antagonismus Atrium -log $K_B$ |
|---|---|---|
| Beispiel 1 | 7,20 | 5,64 |
| Beispiel 9 | 7,93 | 6,30 |
| Beispiel 10 | 8,19 | 5,83 |
| Beispiel 17 | 8,30 | 6,78 |
| Beispiel 18 | 8,43 | 6,90 |
| Beispiel 20 | 8,38 | 7,51 |
| Beispiel 23 | 8,30 | 7,90 |
| Beispiel 30 | 8,21 | 6,68 |
| Beispiel 35 | 7,86 | 7,40 |
| Beispiel 36 | 8,27 | 7,57 |
| Beispiel 37 | 8,13 | 6,91 |
| Beispiel 48 | 7,70 | 7,22 |
| Beispiel 50 | 8,15 | 5,19 |
| Beispiel 55 | 8,14 | 6,43 |
| Beispiel 60 | 8,06 | 6,41 |
| Beispiel 66 | 7,95 | 6,74 |
| Beispiel 70 | 8,02 | 6,87 |
| Beispiel 72 | 7,75 | 7,35 |
| Beispiel 75 | 7,91 | 6,91 |
| Beispiel 79 | 8,61 | 6,61 |
| Beispiel 84 | 8,44 | 6,52 |
| Beispiel 85 | 8,15 | 7,02 |
| Beispiel 88 | 8,74 | 6,66 |
| Beispiel 89 | 8,76 | 6,76 |
| Beispiel 94 | 8,80 | 6,85 |
| Beispiel 96 | 8,51 | 8,28 |
| Beispiel 103 | 7,61 | 5,92 |
| Beispiel 104 | 7,97 | 5,87 |
| Beispiel 105 | 7,44 | 7,54 |

Bevorzugt sind die Arylalkylaminderivate der Formel I, worin $R_1$ für Phenyl, Furyl, Thienyl, Phenyl-$C_1$-$C_3$-alkyl oder (Furyl, Thienyl oder Pyridyl)-$C_1$-$C_3$-alkyl steht, wobei der Phenylring bzw. Heteroarylring jeweils unsubstituiert oder einfach oder zweifach durch Halogen, $C_1$-$C_3$-Alkyl und/oder $C_1$-$C_3$-Alkoxy substituiert ist, oder für Wasserstoff oder $C_1$-$C_3$-Alkyl steht, A ein Stickstoffatom oder eine CH-Gruppe bedeutet, E für die Grup-

pierung $-(CH_2)_p-$, $-O-(CH_2)_p-$, $-S-(CH_2)_p-$ oder

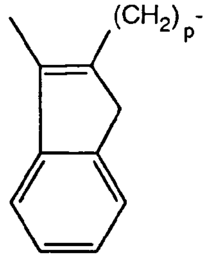

steht, wobei p den Wert 2, 3 oder 4 hat, Q für ein Stickstoffatom oder eine CH-Gruppe steht, $R_2$ Piperidino-$C_1$-$C_3$-alkyl-phenyl, Guanidino-thiazolyl-$C_1$-$C_3$-alkyl, $C_1$-$C_3$-Alkyl-imidazolyl-$C_1$-$C_3$-alkyl oder (N,N-Di-$C_1$-$C_3$-alkylamino-$C_1$-$C_3$-alkyl)-furanyl-$C_1$-$C_3$-alkyl bedeutet, $R_3$ für Wasserstoff oder $C_1$-$C_3$-Alkyl steht, X ein Sauerstoffatom, ein Schwefelatom, die Gruppierung N-CN oder $CH-NO_2$ bedeutet, m den Wert 2, 3, 4, 5, 6, 7 oder 8 und n den Wert 1, 2, 3 oder 4 hat, und Y ein Schwefelatom, ein Sauerstoffatom oder eine Methylengruppe bedeutet, sowie deren stereoisomere Formen, Hydrate und physiologisch annehmbare Salze.

Bei einer bevorzugten Gruppe von erfindungsgemässen Verbindungen der Formel I steht $R_1$ für einen unsubstituierten oder einen einfach oder zweifach, vorzugsweise einfach, mit Halogenatomen, wie Fluor-, Chlor- oder Bromatomen, vorzugsweise Chloratomen, oder mit $C_1$-$C_3$-Alkylgruppen, vorzugsweise Methyl- oder Ethylgruppen, oder mit $C_1$-$C_3$-Alkoxygruppen, wie Methoxy- oder Ethoxygruppen, substituierten Phenylring oder einen Heteroarylring, wie einen Furyl- oder einen Thienylring, bevorzugt einen Phenylring. Im Falle der Einfachsubstitution des durch $R_1$ angegebenen Phenylringes ist der Substituent vorzugsweise in para-Position gebunden, wobei eine para-Chlor-Substitution bevorzugt wird. Im Falle zweifacher Substitution wird die 3,4-, die 3,5- und die 2,4-Disubstitution, insbesondere die 3,4-Disubstitution, bevorzugt. A bedeutet eine CH-Gruppe oder ein Stickstoffatom, Q ein Stickstoffatom oder eine CH-Gruppe. E, $R_2$, $R_3$, X, Y, m, n und p entsprechen der obigen Definition.

Bei einer weiteren bevorzugten Gruppe von erfindungsgemässen Verbindungen der Formel I steht $R_1$ für eine unsubstituierte oder eine einfach oder zweifach, vorzugsweise einfach, mit Halogenatomen, wie Fluor-, Chlor- oder Bromatomen, vorzugsweise Fluor- oder Chloratomen, oder mit $C_1$-$C_3$-Alkylgruppen, vorzugsweise Methyl- oder Ethylgruppen, oder mit $C_1$-$C_3$-Alkoxygruppen wie Methoxy- oder Ethoxygruppen, vorzugsweise Methoxygruppen, substituierte Benzylgruppe oder Heteroarylmethylgruppe, wie eine Furylmethyl-, Thienylmethyl- oder Pyridylmethylgruppe, wobei eine Benzylgruppe bevorzugt wird. Im Falle der Einfachsubstitution der durch $R_1$ angegebenen Benzylgruppe ist der Substituent vorzugsweise in para-Position gebunden, wobei eine para-Fluor- oder para-Methoxy-Substitution bevorzugt wird. Im Falle zweifacher Substitution wird die 3,4-, die 3,5- und die 2,4-Disubstitution, insbesondere die 3,4-Disubstitution, bevorzugt. Q steht für ein Stickstoffatom, A für ein Stickstoffatom und E für die Gruppierung $-CH_2-CH_2-$. $R_2$, $R_3$, X, Y, m, n und p entsprechen der obigen Definition.

Insbesondere bevorzugt sind die Arylalkylaminderivate der Formel I, worin $R_1$ für Phenyl oder Phenyl-$C_1$-$C_3$-alkyl, worin der Phenylring jeweils unsubstituiert durch Halogen, $C_1$-$C_3$-Alkyl oder $C_1$-$C_3$-Alkoxy substituiert ist, oder für Wasserstoff oder $C_1$-$C_3$-Alkyl steht, A ein Stickstoffatom oder eine CH-Gruppe bedeutet, E für die Gruppierung $-(CH_2)_p-$, $-O-(CH_2)_p-$, $-S-(CH_2)_p-$ oder

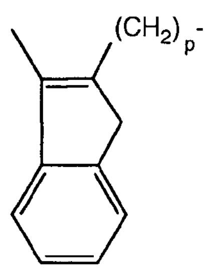

steht, wobei p den Wert 2 oder 3 hat, Q für ein Stickstoffatom oder eine CH-Gruppe steht, $R_2$ Piperidino-$C_1$-$C_3$-alkyl-phenyl, Guanidino-thiazolyl-$C_1$-$C_3$-alkyl, $C_1$-$C_3$-Alkyl-imidazolyl-$C_1$-$C_3$-alkyl oder (N,N-Di-$C_1$-$C_3$-alkylamino-$C_1$-$C_3$-alkyl)-furanyl-$C_1$-$C_3$-alkyl bedeutet, $R_3$ für Wasserstoff oder $C_1$-$C_3$-Alkyl steht, X ein Sauerstoffatom, ein Schwefelatom, die Gruppierung N-CN oder $CH-NO_2$ bedeutet, m den Wert 2, 3, 4, 5, 6, 7 oder 8 und n den Wert 1,2, 3 oder 4 haben kann, und Y ein Schwefelatom oder ein Sauerstoffatom bedeutet, sowie deren stereoisomere Formen, Hydrate und physiologisch annehmbare Salze.

Ganz besonders bevorzugt sind die Arylalkylaminderivate der Formel I, worin $R_1$ für Phenyl, das unsubstituiert oder durch Halogen oder $C_1$-$C_3$-Alkyl substituiert ist, Phenyl-$C_1$-$C_3$-alkyl, das im Phenylring durch Halogen oder $C_1$-$C_3$-Alkoxy substituiert ist, oder für $C_1$-$C_3$-Alkyl steht, A ein Stickstoffatom oder eine CH-Gruppe bedeutet, E für die Gruppierung $-(CH_2)_p-$, $-O-(CH_2)_p-$, $-S-(CH_2)_p-$ oder

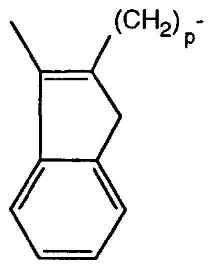

steht, wobei p den Wert 2 hat, Q für ein Stickstoffatom oder eine CH-Gruppe steht, $R_2$ 3-Piperidinomethylphenyl, 2-Guanidino-thiazol-4-ylmethyl, 5-Methyl-imidazol-4-ylmethyl oder 5-(N,N-Dimethylaminomethyl)-furan-2-ylmethyl bedeutet, $R_3$ für $C_1$-$C_3$-Alkyl steht, X ein Sauerstoffatom, ein Schwefelatom, die Gruppierung N-CN oder $CH-NO_2$ bedeutet, m den Wert 2, 3, 4, 5, 6, 7 oder 8 und n den Wert 2, 3 oder 4 haben kann, und Y ein Schwefelatom oder ein Sauerstoffatom bedeutet, sowie deren stereoisomere Formen, Hydrate und physiologisch annehmbare Salze.

Bei einer weiteren bevorzugten Gruppe von erfindungsgemässen Verbindungen der Formel I steht $R_1$ für ein Wasserstoffatom oder eine $C_1$-$C_3$-Alkylgruppe, vorzugsweise eine Methylgruppe, A für eine CH- Gruppe, Q für ein Stickstoffatom und E für die Gruppierung

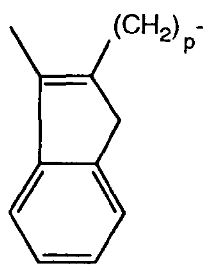

$R_2$, $R_3$, X, Y, m, n und p entsprechen der obigen Definition.

Bei einer bevorzugten Gruppe von erfindungsgemässen Verbindungen der Formel I steht $R_2$ für die Gruppierung 5-Methylimidazol-4-ylmethyl und $R_1$, $R_3$, A, E, Q, X, Y, m, n und p entsprechen der obigen Definition.

Bei einer weiteren bevorzugten Gruppe von erfindungsgemässen Verbindungen der Formel I steht $R_2$ für die Gruppierung

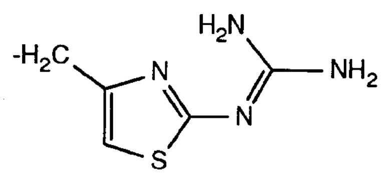

(= 2-Guanidino-thiazol-4-ylmethyl) und $R_1$, $R_3$, A, E, Q, X, Y, m, n und p entsprechen der obigen Definition.

Bei einer weiteren bevorzugten Gruppe von erfindungsgemässen Verbindungen der Formel I steht $R_2$ für die Gruppierung 5-(N,N-Dimethylaminomethyl)-furan-2-ylmethyl und $R_1$, $R_3$, A, E, Q, X, Y, m, n und p entsprechen der obigen Definition.

Bei einer weiteren bevorzugten Gruppe von erfindungsgemässen Verbindungen der Formel I steht $R_2$ für die Gruppierung 3-Piperidinomethyl-phenyl und $R_1$, $R_3$, A, E, Q, X, Y, m, n und p entsprechen der obigen Definition.

Die Erfindung umfasst auch alle stereoisomeren Formen und Hydrate der oben beschriebenen Verbindungen der allgemeinen Formel I.

Die Erfindung betrifft vor allem die in den Beispielen beschriebenen spezifischen Verbindungen und Salze, insbesondere pharmazeutisch verwendbare Salze, davon.

Die erfindungsgemässen Verbindungen können in an sich bekannter Weise z.B. dadurch hergestellt werden, dass man

a) zur Herstellung einer Verbindung der Formel I, in der X für ein Sauerstoff- oder ein Schwefelatom, die Gruppierung N-CN oder $CH-NO_2$ steht,

(a1) eine Verbindung der Formel II,

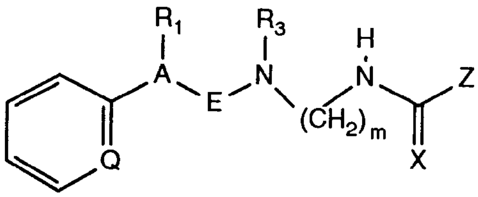

(II)

in der $R_1$, $R_3$, A, E, Q und m die oben genannte Bedeutung besitzen und Z für eine Methylthio-, eine Mercapto- oder eine Phenoxygruppe steht, mit einer Verbindung der allgemeinen Formel III,

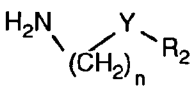

(III)

in der $R_2$, Y und n die oben angegebene Bedeutung besitzen, vorzugsweise in äquimolaren Mengen in einem polaren Lösungsmittel, wie einem Alkohol, zum Beispiel Methanol, Ethanol, oder Isopropylalkohol, oder in Acetonitril, Dimethylsulfoxid, Dimethylformamid, Pyridin, vorzugsweise Acetonitril, z.B. bei Raum- oder Rückflusstemperatur des entsprechenden Lösungsmittels, umsetzt, oder

(a2) eine Verbindung der Formel IV,

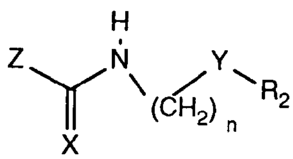

(IV)

in der $R_2$, Y und n die oben angegebene Bedeutung besitzen und Z für eine Methylthio-, eine Mercapto- oder eine Phenoxygruppe steht, vorzugsweise in äquimolaren Mengen, mit einer Verbindung der allgemeinen Formel V,

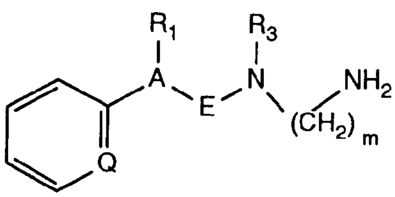

(V)

in der $R_1$, $R_3$, A, E, Q und m die in Anspruch 1 genannte Bedeutung besitzen, vorzugsweise in einem polaren Lösungsmittel wie oben erwähnt, umsetzt, oder

b) zur Herstellung einer Verbindung der Formel I, in der X für ein Sauerstoff- oder ein Schwefelatom steht,

(b1) eine Verbindung der Formel VI,

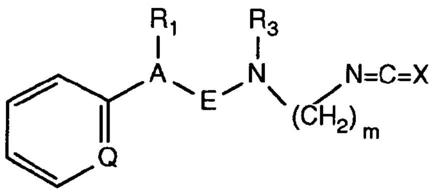

(VI)

bei der $R_1$, $R_3$, A, E, Q und m die oben genannte Bedeutung besitzen, vorzugsweise in äquimolaren Mengen, mit einer Verbindung der Formel III, in der $R_2$, Y und n die oben genannte Bedeutung besitzen, vorzugsweise in einem inerten Lösungsmittel, z.B. Dimethylformamid oder einem Ether, insbesondere Tetrahydrofuran, z.B. bei Raum- oder Rückflusstemperatur des entsprechenden Lösungsmittels, umsetzt, oder

(b2) eine Verbindung der Formel VII,

$$X = C = N - (CH_2)_n - Y - R_2 \qquad \text{(VII)}$$

bei der $R_2$, Y und n die oben genannte Bedeutung besitzen, vorzugsweise in äquimolaren Mengen, mit einer Verbindung der Formel V,

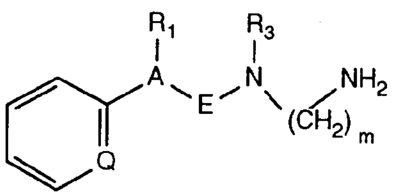

(V)

bei der $R_1$, $R_3$, A, E, Q und m die oben genannte Bedeutung besitzen, vorzugsweise in einem inerten Lösungsmittel wie oben erwähnt, z.B. Dimethylformamid oder einem Ether, insbesondere Tetrahydrofuran, z.B. bei Raum- oder Rückflusstemperatur des entsprechenden Lösungsmittels, umsetzt;

und, wenn erwünscht, eine erhaltene Verbindung der Formel I in eine andere Verbindung der Formel I umwandelt, und/oder, wenn erwünscht, ein erhaltenes Salz in die freie Verbindung oder in ein anderes Salz umwandelt, und/oder, wenn erwünscht, eine erhaltene freie Verbindung der Formel I mit salzbildenden Eigenschaften in ein Salz, insbesondere ein physiologisch annehmbares Salz, umwandelt.

Gegebenenfalls werden die nach den Verfahren a) oder b) erhaltenen Verbindungen in an sich bekannter Weise chromatographiert und/oder anderweitig, z.B. durch Umkristallisieren, gereinigt.

Die Erfindung umfasst neben den stereoisomeren Verbindungen und Hydraten der Substanzen der allgemeinen Formel I auch die physiologisch annehmbaren Salze dieser Verbindungen. Diese Salze können zum Beispiel mit Mineralsäuren, wie Chlor-, Brom-, Iodwasserstoffsäure, Phosphorsäure, Metaphosphorsäure, Salpetersäure oder Schwefelsäure, oder mit organischen Säuren, wie z.B. Ameisensäure, Essigsäure, Propionsäure, Phenylessigsäure, Weinsäure, Zitronensäure, Fumarsäure, Methansulfonsäure oder Embonsäure, gebildet werden.

Verfahrensgemäss erhältliche freie Verbindungen der Formel I mit salzbildenden Eigenschaften können in an sich bekannter Weise in ihre Salze überführt werden, Verbindungen mit basischen Eigenschaften z.B. durch Behandeln mit Säuren oder geeigneten Derivaten davon.

Infolge der engen Beziehung zwischen den Verbindungen der Formel I in freier Form und in Form von Salzen sind vor- und nachstehend unter den freien Verbindungen bzw. ihren Salzen sinn- und zweckmässig gegebenenfalls auch die entsprechenden Salze bzw. freien Verbindungen zu verstehen.

Die oben angeführten Reaktionen können unter an sich bekannten Reaktionsbedingungen durchgeführt werden, in Ab- oder üblicherweise Anwesenheit von Lösungs- oder Verdünnungsmitteln, vorzugsweise solchen, die gegenüber den verwendeten Reagenzien inert sind und diese lösen, in Ab- oder Anwesenheit von Katalysatoren, Kondensationsmitteln oder neutralisierenden Agentien, je nach Art der Reaktion und/oder der Reaktionsteilnehmer bei erniedrigter, normaler oder erhöhter Temperatur, z.B. im Temperaturbereich von etwa -70°C bis etwa 190°C, vorzugsweise von etwa -20°C bis etwa 150°C, z.B. beim Siedepunkt des verwendeten Lösungsmittels, unter atmosphärischem Druck oder,in einem geschlossenen Gefäss, gegebenenfalls unter Druck, und/oder in einer inerten Atmosphäre, z.B. unter einer Stickstoffatmosphäre.

Im Verfahren der vorliegenden Erfindung werden vorzugsweise solche Ausgangsstoffe eingesetzt, die zu den eingangs als besonders wertvoll beschriebenen Verbindungen führen.

Die Erfindung betrifft auch diejenigen Ausführungsformen des Verfahrens, bei denen man von einer auf einer beliebigen Verfahrensstufe als Zwischenprodukt erhältlichen Verbindung ausgeht und die fehlenden Verfahrensschritte durchführt, oder bei denen ein Ausgangsstoff unter den Reaktionsbedingungen gebildet oder in Form eines Derivates, z.B. eines Salzes davon, verwendet wird.

Die erfindungsgemässen Verbindungen können zur Verabreichung in jeder beliebigen Weise formuliert werden. Die Erfindung umfasst daher auch pharmazeutische Präparate, die mindestens eine erfindungsgemässe Verbindung zur Verwendung in der Human- oder Veterinärmedizin enthalten. Solche Arzneimittel können unter Verwendung von einem oder mehreren pharmazeutischen Trägem oder Verdünnungsmitteln hergestellt werden.

Die erfindungsgemässen Verbindungen können daher z.B. für die orale, bukkale, topische, parenterale oder rektale Verabreichung formuliert werden, wobei die orale Verabreichung bevorzugt wird.

Für die bukkale Verabreichung kann das Arzneimittel z.B. in Form von Tabletten oder Briefchen vorliegen, die in herkömmlicher Weise formuliert worden sind.

Die erfindungsgemässen Verbindungen können für die parenterale Verabreichung z.B. durch Bolusinjektion oder kontinuierliche Infusion formuliert werden. Formulierungen zur Injektion können z.B. in Dosiseinheitsform als Ampullen oder in Mehrfachdosenbehältern mit zugesetztem Konservierungsmittel vorliegen.

Die Arzneimittel können z.B. auch als Suspensionen, Lösungen oder Emulsionen in öligen oder wässrigen Trägern, vorliegen, und sie können Formulierungshilfsmittel, wie z.B. Suspendierungs-, Stabilisierungs- und/oder Dispergierungsmittel, enthalten.

Alternativ kann der Wirkstoff z.B. auch in Pulverform zur Rekonstitution mit einem geeigneten Träger, zum Beispiel sterilem, pyrogenfreiem Wasser, vor dem Gebrauch vorliegen.

Die erfindungsgemässen Verbindungen können z.B. auch als rektale Zubereitungen, z.B. Suppositorien oder Retentionseinläufe, formuliert werden, die z.B. Suppositoriengrundlagen, wie Kakaobutter oder andere Glyceride, enthalten.

Zur topischen Anwendung können die erfindungsgemässen Verbindungen z.B. als Salben, Crèmes, Gele, Lotionen, Pulver oder Sprays formuliert werden.

Für die orale Verabreichung an einen Warmblüter mit etwa 70 kg Körpergewicht ist eine geeignete Tagesdosis an erfindungsgemässen Verbindungen 5 mg bis 1 g, vorzugsweise 5 bis 250 mg, je nach Zustand des Patienten, verteilt auf z.B. 1 bis 4 Einzeldosen. Im Einzelfall kann es gegebenenfalls erforderlich sein, von den genannten Mengen abzuweichen, und zwar in Abhängigkeit vom individuellen Verhalten gegenüber dem Wirkstoff bzw. der Art seiner Formulierung und dem Zeitpunkt bzw. Intervall, zu welchem die Verabreichung erfolgt. So gibt es zum Beispiel Fälle, wo mit weniger als der oben genannten Mindestmenge ausgekommen werden kann, während in anderen Fällen die genannte obere Grenze überschritten werden muss.

Die nachfolgenden Beispiele illustrieren die vorliegende Erfindung; "furfuryl" steht für "furan-2-ylmethyl".

Beispiel 1: N-[2-[N-[3-(4-Chlorphenyl)-3-(2-pyridyl)propyl]-N-methylamino]ethyl]-N′-cyano-N″-[4-[3-(piperidinomethyl)phenoxy]butyl]guanidin

Eine Mischung aus 0,8 g (2,6 mmol) N-[3-(4-Chlorphenyl)-3-(2-pyridyl)propyl]-N-methyl -1,2-ethandiamin (A) und 0,8 g (2 mmol) N-Cyano-O-phenyl-N′-[4-[3-(piperidinomethyl)phenoxy]butyl]isoharnstoff (B) wird in 20 ml absolutem Acetonitril 12 Stunden unter Rückfluss erhitzt. Danach wird der Ansatz im Vakuum vom Lösungsmittel befreit und das Reaktionsprodukt mittels präparativer Dickschichtchromatographie isoliert (Kieselgel 60 PF254; Ethylacetat/ Methanol 95+5, Ammoniakatmosphäre). Man erhält die Titelverbindung als zähes Öl; MS (+FAB-Methode): m/z ( rel. Int. [%]) = 616 ($[M+H]^+$, 9), 230 (100); IR (KBr): 2163 $cm^{-1}$ (C≡N). Eine Probe wird zu analytischen Zwecken in das Salz der O,O′-Ditoluoylweinsäure überführt und aus Ether/Isopropanol umkristallisiert; Smp.: ab 135 °C Zersetzung.

Die Ausgangsverbindungen werden wie folgt hergestellt:

(A) Eine Lösung von 3-(4-Chlorphenyl)-3-(2-pyridyl)propanamin [A. Buschauer, Arch. Pharm. (Weinheim) 322, 165-171 (1989)] (0,15 mol) in 100 ml Ether wird mit 100 ml 10 %iger NaOH unterschichtet und unter Eiskühlung kräftig gerührt. Es wird Chlorameisensäureethylester langsam zugetropft, bis an der Eintropfstelle keine Fällung mehr auftritt. Die organische Phase wird mit Wasser neutralgewaschen, über $Na_2SO_4$ getrocknet, im Vakuum eingedampft und mittels längerer Evakuierung im Ölpumpenvakuum intensiv getrocknet. Das resultierende Öl ist für die weitere Umsetzung rein genug. 0,12 mol des Reaktionsproduktes wird in 100 ml absolutem THF gelöst und in eine gerührte und eisgekühlte Suspension von 9,4 g (0,24 mol) $LiAlH_4$ in 150 ml THF getropft und noch 2 Stunden unter Rückfluss erhitzt. Dem Ansatz wird nach Abkühlen und Versetzen mit wassergesättigtem Ether ca. 10 ml 10 %iger NaOH zugesetzt und über Nacht gerührt. Die Fällung wird abgesaugt und das klare Filtrat im Vakuum eingedampft. Das Rohprodukt von 3-(4-Chlorphenyl)-3-(2-pyridyl)-N-methyl-1-propanamin [analog in A. Buschauer, J. Med. Chem. 32, 1963 (1989)] wird nach säulenchromatographischer Reinigung (Ethylacetat/ammoniak. Methanol 99+1) in einer

Ansatzgrösse von 10-30 mmol mit einer äquivalenten Menge Chloracetonitril, einer Spatelspitze Kaliumiodid und einem doppelt molaren Überschuss an $Na_2CO_3$ in 20 ml einer Mischung aus gleichen Anteilen Acetonitril und DMF 2 Stunden bei 60°C gerührt. Nach Ende der Reaktion (dc-Kontrolle, FM VI) wird der Ansatz mit 20 ml Wasser versetzt und mehrmals mit Toluol extrahiert. Die vereinigten organischen Extrakte werden mit Wasser gewaschen, über $Na_2SO_4$ getrocknet und mit einer kleinen Menge Kieselgel für die Säulenchromatographie (SC) entfärbt. Nach Eindampfen im Vakuum erhält man das Reaktionsprodukt, 2-[N-[3-(4-Chlorphenyl)-3-(2-pyridyl)propyl]-N-methylamino]-acetonitril in genügender Reinheit mit einer Ausbeute von ca. 80 %. Das so dargestellte Nitril wird in Ether gelöst und in eine mit Eis gekühlte und gerührte Suspension von $LiAlH_4$ (1,5 Äquivalente) in absolutem Ether (Gesamtvolumen ca. 50 ml) getropft und 2 Stunden bei Raumtemperatur belassen. Nach vollständiger Umsetzung des Eduktes wird der Ansatz mit wassergesättigtem Ether und 2-3 ml 10 %iger NaOH versetzt und über Nacht bei Raumtemperatur gerührt. Nach Absaugen der Anorganica wird das Filtrat im Vakuum eingedampft und das resultierende Amin bei genügender Reinheit weiter umgesetzt oder mittels präparativer Dickschichtchromatographie isoliert.

(B) 4,6 g (0,2 mol) Natrium werden in wasserfreiem Ethanol gelöst und mit 38,24 g (0,2 mol) 3-Piperidinomethylphenol [DE-A-2917026, Glaxo] versetzt. Nach Zutropfen von 20,71 g (0,2 mol) 4-Chlorbutyronitril in Ethanol wird unter Feuchtigkeitsausschluss über Nacht unter Rückfluss erhitzt. Anschliessend lässt man den Ansatz abkühlen, filtriert und engt im Vakuum zur Trockne ein. Das resultierende Öl wird in Ether aufgenommen und mit 10 %iger NaOH und Wasser gewaschen. Nach Trocknen über $Na_2SO_4$ und Einengen werden 35,5 g (0,14 mol) 4-(3-Piperidinomethylphenoxy)butyronitril als Öl erhalten, das ohne weitere Reinigung in die nachfolgende Reaktion eingesetzt werden kann. 59,9 mmol 4-(3-Piperidinomethylphenoxy)butyronitril werden unter Eiskühlung und Rühren langsam in eine Suspension von 2,98 g (78,4 mmol) $LiAlH_4$ in 140 ml wasserfreiem Ether eingetragen. Nach 40 min Rühren bei Raumtemperatur wird der Ansatz unter Eiskühlung mit 10 ml wassergesättigtem Ether und 7 ml 10 %iger NaOH hydrolysiert. Man lässt weitere 30 min Rühren, saugt dann ab und wäscht den Niederschlag mehrmals mit Ether nach. Das Filtrat wird mit Wasser gewaschen, über $Na_2SO_4$ getrocknet, filiert und im Vakuum bis zur Trockne eingeengt. Es resultiert das 4-(3-Piperidinomethylphenoxy)butanamin als dünnflüssiges, braunes Öl, das ohne weitere Reinigung in die nachfolgende Reaktion eingesetzt werden kann.Zu einer Suspension von N-Cyanodiphenylimidocarbonat in 100 ml Diethylether wird unter Rühren die äquimolare Menge 4-(3-Piperidinomethylphenoxy)butanamin zugegeben. Nach ca. 15 min Rühren kommt es zur massiven Fällung des Produktes N-Cyano-O-phenyl-N′-[4-(3-piperidinomethylphenoxy)butyl]isoharnstoff, oder es kristallisiert nach Einengen der Lösung. Man saugt ab, wäscht mit Diethylether und trocknet.

Beispiel 2: N-[5-[N-[3-(4-Chlorphenyl)-3-(2-pyridyl)propyl]-N-methylamino]pentyl]-N′-cyano-N″-[3-[3-(piperidinomethyl)phenoxy]propyl]guanidin

Die Herstellung erfolgt analog Beispiel 1 ausgehend von 1,09 g (3,15 mmol) N-[3-(4-Chlorphenyl)3-(2-pyridyl)propyl]-N-methyl-1,5-pentandiamin und 1,23 g (3,1 mmol) N-Cyano-O-phenyl-N′-[3-[3-(piperidinomethyl)phenoxy]propyl]isoharnstoff. Nach chromatographischer Aufarbeitung analog Beispiel 1 erhält man die gereinigte Titelverbindung als zähes Öl; IR (KBr): 2163 $cm^{-1}$ (C≡N).

Beispiel 3: N-[4-[N-[3-(4-Chlorphenyl)-3-(2-pyridyl)propyl]-N-methylamino]butyl]-N′-cyano-N″-[3-[3-(piperidinomethyl)phenoxy]propyl]guanidin

Die Herstellung erfolgt analog Beispiel 1 ausgehend von 0,99 g (3 mmol) N-[3-(4-Chlorphenyl)-3-(2-pyridyl)propyl]-N-methyl-1,4-butandiamin und 1,17 g (3 mmol) N-Cyano-O-phenyl-N′-[3-[3-(piperidinomethyl-)phenoxy]propyl]isoharnstoff. Nach chromatographischer Aufarbeitung (Eluens: Methylenchlorid/ Methanol 98+2) analog Beispiel 1 erhält man die gereinigte Titelverbindung als zähes Öl; MS (+FAB-Methode): m/z ( rel. Int.[%]) = 630 ($[M+H]^+$, 7), 230 (100); IR (KBr): 2164 $cm^{-1}$ (C≡N).

Beispiel 4: N-[3-[N-[3-(4-Chlorphenyl)-3-(2-pyridyl)propyl]-N-methylamino]propyl]-N′-cyano-N″-[3-[3-(piperidinomethyl)phenoxy]propyl]guanidin

Die Herstellung erfolgt analog Beispiel 1 ausgehend von 0,5 g (1,57 mmol) N-[3-(4-Chlorphenyl)-3-(2-pyridyl)propyl]-N-methyl-1,3-propandiamin und 0,6 g (1,5 mmol) N-Cyano-O-phenyl-N′-[3-[3-(piperidinomethyl)phenoxy]propyl]isoharnstoff. Nach chromatographischer Aufarbeitung (Eluens: Ethylacetat) analog Beispiel 1 erhält man die gereinigte Titelverbindung als zähes Öl; MS (+FAB-Methode): m/z ( rel. Int.[%]) = 616 ($[M+H]^+$, 1), 230 (100).

Beispiel 5: N-[2-[N-[3-(4-Chlorphenyl)-3-(2-pyridyl)propyl]-N-methylamino]ethyl]-N′-cyano-N″-[3-[3-(piperidinomethyl)phenoxy]propyl]guanidin

Die Herstellung erfolgt analog Beispiel 1 ausgehend von 0,7 g (2,3 mmol) N-[3-(4-Chlorphenyl)-3-(2-pyridyl)propyl]-N-methyl-1,2-ethandiamin und 0,85 g (2,2 mmol) N-Cyano-O-phenyl-N′-[3-[3-(piperidinomethyl)phenoxy]propyl]isoharnstoff. Nach chromatographischer Aufarbeitung (Eluens: Ethylacetat) analog Beispiel 1 erhält man die gereinigte Titelverbindung als zähes Öl; MS (+FAB-Methode): m/z ( rel. Int.[%]) = 602 ([M+H]$^+$, 5), 230 (100); IR (KBr): 2165 cm$^{-1}$ (C≡N). Eine Probe wird zu analytischen Zwecken in das Salz der O,O′-Ditoluoylweinsäure überführt und aus Ether/Isopropanol umkristallisiert; Smp.: ab 85°C Zersetzung.

Beispiel 6: N-Cyano-N′-[4-[N-[3-[4-fluorphenyl)-3-(2-pyridyl)propyl]-N-methylamino]-butyl]-N″-[3-[3-(piperidinomethyl)phenoxy]propyl]guanidin

Die Herstellung erfolgt analog Beispiel 1 ausgehend von 0,8 g (2,5 mmol) N-[3-(4-Fluorphenyl)-3-(2-pyridyl)propyl]-N-methyl-1,4-butandiamin und 0,99 g (2,5 mmol) N-Cyano-O-phenyl-N′-[3-[3-(piperidinomethyl)phenoxy]propyl]isoharnstoff. Nach chromatographischer Aufarbeitung analog Beispiel 1 erhält man die gereinigte Titelverbindung als zähes Öl; MS (+FAB -Methode): m/z ( rel. Int. [%]) = 614 ([M+H]$^+$, 3), 214 (50,2), 154 ([m-$NO_2$-BenzylOH], 100); IR (KBr): 2163 cm$^{-1}$ (C≡N). Eine Probe wird zu analytischen Zwecken in das Salz der O,O′-Ditoluoylweinsäure überführt und aus Ether/Isopropanol umkristallisiert; Smp.: 105-110°C Zersetzung.

Beispiel 7: N-Cyano-N′-[3-[N-[3-(4-fluorphenyl)-3-(2-pyridyl)propyl]-N-methylamino]propyl]-N″-[3-[3-(piperidinomethyl)phenoxy]propyl]guanidin

Die Herstellung erfolgt analog Beispiel 1 ausgehend von 0,8 g (2,7 mmol) N-[3-(4-Fluorphenyl)-3-(2-pyridyl)propyl]-N-methyl-1,3-propandiamin und 1,04 g (2,6 mmol) N-Cyano-O-phenyl-N′-[3-[3-(piperidinomethyl)phenoxy]propyl]isoharnstoff. Nach chromatographischer Aufarbeitung analog Beispiel 1 erhält man die gereinigte Titelverbindung als zähes Öl; MS (+FAB-Methode): m/z ( rel. Int.[%]) = 600 ([M+H]$^+$ 22,4), 214 (100); IR (KBr): 2164 cm$^{-1}$ (C≡N).

Beispiel 8: N-Cyano-N′-[2-[N-[3-(4-fluorphenyl)-3-(2-pyridyl)propyl]-N-methylamino]ethyl]-N″-[3-[3-(piperidinomethyl)phenoxy]propyl]guanidin

Die Herstellung erfolgt analog Beispiel 1 ausgehend von 0,7 g (2,4 mmol) N-[3-(4-Fluorphenyl)-3-(2-pyridyl)propyl]-N-methyl-1,2-ethandiamin und 0,95 g (2,4 mmol) N-Cyano-O-phenyl-N′-[3-[3-(piperidinomethyl)phenoxy]propyl]isoharnstoff. Nach chromatographischer Aufarbeitung analog Beispiel 1 erhält man die gereinigte Titelverbindung als zähes Öl; MS (+FAB-Methode): m/z ( rel. Int.[%]) = 586 ([M+H]$^+$, 1), 214 (100).

Beispiel 9: N-Cyano-N′-[4-[N-[3-phenyl-3-(2-pyridyl)propyl]-N-methylamino]butyl]-N″-[3-[3-(piperidinomethyl)phenoxy]propyl]guanidin

Die Herstellung erfolgt analog Beispiel 1 ausgehend von 0,7 g (2,3 mmol) N-Methyl-N-[3-phenyl-3-(2-pyridyl)propyl]-1,4-butandiamin und 0,89 g (2,3 mmol) N-Cyano-O-phenyl-N′-[3-[3-(piperidinomethyl)phenoxy]propyl]isoharnstoff. Nach chromatographischer Aufarbeitung analog Beispiel 1 erhält man die gereinigte Titelverbindung als zähes Öl; MS (+FAB-Methode): m/z ( rel. Int.[%]) = 596 ([M+H]$^+$ 7), 196 (100).

Beispiel 10: N-Cyano-N′-[3-[N-[3-phenyl-3-(2-pyridyl)propyl]-N-methylamino]propyl]-N″-[3-[3-(piperidinomethyl)phenoxy]propyl]guanidin

Die Herstellung erfolgt analog Beispiel 1 ausgehend von 0,57 g (2 mmol) N-Methyl-N-[3-phenyl-3-(2-pyridyl)propyl]-1,3-propandiamin und der äquimolaren Menge N-Cyano-O-phenyl-N′-[3-[3-(piperidinomethyl)phenoxy]propyl]isoharnstoff. Nach chromatographischer Aufarbeitung analog Beispiel erhält man die gereinigte Titelverbindung als zähes Öl; IR (KBr): 2164 cm$^{-1}$ (C≡N).

Beispiel 11: N-Cyano-N′-[2-[N-[3-phenyl-3-(2-pyridyl)propyl]-N-methylamino]ethyl]-N″-[3-[3-(piperidinomethyl)phenoxy]propyl]guanidin

Die Herstellung erfolgt analog Beispiel 1 ausgehend von 0,7 g (2,6 mmol) N-Methyl-N-[3-phenyl-3-(2-pyridyl)propyl]-1,2-ethandiamin und 1g (2,5 mmol) N-Cyano-O-phenyl-N′-[3-[3-(piperidinomethyl)phenoxy]propyl]isoharnstoff. Nach chromatographischer Aufarbeitung (Eluens: Ethylacetat) analog Beispiel 1 erhält man die gereinigte Titelverbindung als zähes Öl; MS (+FAB-Methode): m/z ( rel. Int.[%]) = 568 ($[M+H]^+$ 3), 196 (100).

Beispiel 12: N-Cyano-N′-[2-[[(2-guanidino-4-thiazolyl)methyl]thio]ethyl]-N″-[2-[N-[3-phenyl-3-(2-pyridyl)propyl]-N-methylamino]ethyl]guanidin

Die Herstellung erfolgt analog Beispiel 1 ausgehend von 0,8 g (3 mmol) N-Methyl-N-[3-phenyl-3-(2-pyridyl)propyl]-1,2-ethandiamin und 1,01 g (2,7 mmol) N-Cyano-N′-[2-[[(2-guanidino-4-thiazolyl)methyl]thio]ethyl]-O-phenyl-isoharnstoff. Nach chromatographischer Aufarbeitung analog Beispiel 1 erhält man die gereinigte Titelverbindung als zähes Öl; MS (+FAB-Methode): m/z ( rel. Int.[%]) = 551 ($[M+H]^+$, 2), 196 (100); IR (KBr): 2164 $cm^{-1}$ (C≡N). Eine Probe wird zu analytischen Zwecken in das Salz der Maleinsäure überführt und aus Ether/ Ethanol umkristallisiert; Smp.: 115-116°C Zersetzung.

Beispiel 13: N-Cyano-N′-[2-[[(2-guanidino-4-thiazolyl)methyl]thio]ethyl]-N″-[3-[N-[3-phenyl-3-(2-pyridyl)propyl]-N-methylamino]propyl]guanidin

Die Herstellung erfolgt analog Beispiel 1 ausgehend von 0,85 g (3 mmol) N-Methyl-N-[3-phenyl-3-(2-pyridyl)propyl]-1,3-propandiamin und 1,12 g (3 mmol) N-Cyano-N′-[2-[[(2-guanidino-4-thiazolyl)methyl]thio]ethyl]-O-phenyl-isoharnstoff. Nach chromatographischer Aufarbeitung analog Beispiel 1 erhält man die gereinigte Titelverbindung als Trockenschaum; MS (+FAB-Methode): m/z ( rel. Int.[%]) = 565 ($[M+H]^+$, 2), 196 (100); IR (KBr): 2162 $cm^{-1}$ (C≡N). Eine Probe wird zu analytischen Zwecken in das Salz der Maleinsäure überführt und aus Ether/ Isopropanol umkristallisiert; Smp.: 110-112°C Zersetzung.

Beispiel 14: N-Cyano-N′-[4-[N-[3-(4-fluorphenyl)-3-(2-pyridyl)propyl]-N-methylamino]-butyl]-N″-[2-[[(2-guanidino-4-thiazolyl)methyl]thio]ethyl]guanidin

Die Herstellung erfolgt analog Beispiel 1 ausgehend von 0,8 g (2,5 mmol) N-[3-(4-Fluorphenyl)-3-(2-pyridyl)propyl]-N-methyl-1,4-butandiamin und 0,95 g (2,5 mmol) N-Cyano-N′-[2-[[(2-guanidino-4-thiazolyl)methyl]thio]ethyl]-O-phenyl-isoharnstoff. Nach chromatographischer Aufarbeitung (Eluens: Methylenchlorid/ Methanol 95+5) analog Beispiel 1 erhält man die gereinigte Titelverbindung als zähes Öl; MS (+FAB-Methode): m/z ( rel. Int.[%]) = 597 ($[M+H]^+$ 1), 214 (100); IR (KBr): 2162 $cm^{-1}$ (C≡N).

Beispiel 15: N-[2-[N-[3-(4-Chlorphenyl)-3-(2-pyridyl)propyl]-N-methylamino]ethyl]-N′-cyano-N″-[2-[[(2-guanidino-4-thiazolyl)methyl]thio]ethyl]guanidin

Die Herstellung erfolgt analog Beispiel 1 ausgehend von 0,8 g (2,6 mmol) N-[3-(4-Chlorphenyl)-3-(2-pyridyl)propyl]-N-methyl-1,2-ethandiamin und 0,98 g (2,6 mmol) N-Cyano-N′-[2-[[(2-guanidino-4-thiazolyl)methyl]thio]ethyl]-O-phenyl-isoharnstoff. Nach chromatographischer Aufarbeitung analog Beispiel 1 erhält man die gereinigte Titelverbindung als Trockenschaum; MS (+FAB-Methode): m/z ( rel. Int.[%]) = 585 ($[M+H]^+$, 6), 230 (100); IR (KBr): 2164 $cm^{-1}$ (C≡N). Eine Probe wird zu analytischen Zwecken in das Salz der O,O′-Ditoluoylweinsäure überführt und aus Ether/Isopropanol umkristallisiert; Smp.: ab 160°C Zersetzung.

Beispiel 16: N-[3-[N-[3-(4-Chlorphenyl)-3-(2-pyridyl)propyl]-N-methylamino]propyl]-N′-cyano-N″-[2-[[(2-guanidino-4-thiazolyl)methyl]thio]ethyl]guanidin

Die Herstellung erfolgt analog Beispiel 1 ausgehend von 0,8 g (2,5 mmol) N-[3-(4-Chlorphenyl)-3-(2-pyridyl)propyl]-N-methyl-1,3-propandiamin und 0,94 g (2,5 mmol) N-Cyano-N′-[2-[[(2-guanidino-4-thiazolyl)methyl]thio]ethyl]-O-phenyl-isoharnstoff. Nach chromatographischer Aufarbeitung analog Beispiel 1 erhält man die gereinigte Titelverbindung als Trockenschaum; MS (+FAB-Methode): m/z ( rel. Int.[%]) = 599 ($[M+H]^+$, 7), 230 (100); IR (KBr): 2161 $cm^{-1}$ (C≡N). Eine Probe wird zu analytischen Zwecken in das Salz der O,O′-Ditoluoylweinsäure überführt und aus Ether/Isopropanol umkristallisiert; Smp.: ab 160°C Zersetzung.

Beispiel 17: N-[4-[N-[3-(4-Chlorphenyl)-3-(2-pyridyl)propyl]-N-methylamino]butyl]-N′-cyano-N″-[2-[[(2-guanidino-4-thiazolyl)methyl]thio]ethyl]guanidin

Die Herstellung erfolgt analog Beispiel 1 ausgehend von 0,7 g (2,1 mmol) N-[3-(4-Chlorphenyl)-3-(2-pyridyl)propyl]-N-methyl-1,4-butandiamin und 0,79 g (2,1 mmol) N-Cyano-N′-[2-[[(2-guanidino-4-thiazolyl)methyl]thio]ethyl]-O-phenyl-isoharnstoff. Nach chromatographischer Aufarbeitung analog Beispiel 1 erhält man die gereinigte Titelverbindung als Trockenschaum; MS (+FAB-Methode): m/z ( rel. Int.[%]) = 613 ($[M+H]^+$, 2), 230 (100).

Beispiel 18: N-[5-[N-[3-(4-Chlorphenyl)-3-(2-pyridyl)propyl]-N-methylamino]pentyl]-N′-cyano-N″-[2-[[(2-guanidino-4-thiazolyl)methyl]thio]ethyl]guanidin

Die Herstellung erfolgt analog Beispiel 1 ausgehend von 0,75 g (2,1 mmol) N-[3-(4-Chlorphenyl)-3-(2-pyridyl)propyl]-N-methyl-1,5-pentandiamin und 0,81 g (2,1 mmol) N-Cyano-N′-[2-[[(2-guanidino-4-thiazolyl)methyl]thio]ethyl]-O-phenyl-isoharnstoff. Nach chromatographischer Aufarbeitung analog Beispiel 1 erhält man die gereinigte Titelverbindung als Trockenschaum; MS (+FAB-Methode): m/z ( rel. Int.[%]) = 627 ($[M+H]^+$, 3), 230 (100); IR (KBr): 2162 $cm^{-1}$ (C≡N). Eine Probe wird zu analytischen Zwecken in das Salz der Maleinsäure überführt und aus Ether/ Ethanol umkristallisiert; Smp.: 87-90°C.

Beispiel 19: N-[6-[N-[3-(4-Chlorphenyl)-3-(2-pyridyl)propyl]-N-methylamino]hexyl]-N′-cyano-N″-[2-[[(2-guanidino-4-thiazolyl)methyl]thio]ethyl]guanidin

Die Herstellung erfolgt analog Beispiel 1 ausgehend von 0,5 g (1,4 mmol) N-[3-(4-Chlorphenyl)-3-(2-pyridyl)propyl]-N-methyl-1,6-hexandiamin und 0,52 g (1,4 mmol) N-Cyano-N′-[2-[[(2-guanidino-4-thiazolyl)methyl]thio]ethyl]-O-phenyl-isoharnstoff. Nach chromatographischer Aufarbeitung (Eluens: Methylenchlorid/ Methanol 95+5) analog Beispiel 1 erhält man die gereinigte Titelverbindung als Trockenschaum; MS (+FAB-Methode): m/z ( rel. Int.[%]) = 641 ($[M+H]^+$, 5), 230 (47), 154 (100); IR (KBr): 2161 $cm^{-1}$ (C≡N).

Beispiel 20: N-[7-[N-[3-(4-Chlorphenyl)-3-(2-pyridyl)propyl]-N-methylamino]heptyl]-N′-cyano-N″-[2-[[(2-guanidino-4-thiazolyl)methyl]thio]ethyl]guanidin

Die Herstellung erfolgt analog Beispiel 1 ausgehend von 0,7 g (1,9 mmol) N-[3-(4-Chlorphenyl)-3-(2-pyridyl)propyl]-N-methyl-1,7-heptandiamin und 0,7 g (1,9 mmol) N-Cyano-N′-[2-[[(2-guanidino-4-thiazolyl)methyl]thio]ethyl]-O-phenyl-isoharnstoff. Nach chromatographischer Aufarbeitung (Eluens: Methylenchlorid/ Methanol 95+5) analog Beispiel 1 erhält man die gereinigte Titelverbindung als Trockenschaum; MS (+FAB-Methode): m/z ( rel. Int.[%]) = 655 ($[M+H]^+$ 2), 230 (18), 154 (100); IR (KBr): 2161 $cm^{-1}$ (C≡N). Eine Probe wird zu analytischen Zwecken in das Salz der O,O′-Ditoluoylweinsäure überführt und aus Ether/ Isopropanol umkristallisiert; Smp.: ab 150°C Zersetzung.

Beispiel 21: N-Cyano-N′-[2-[[(2-guanidino-4-thiazolyl)methyl]thio]ethyl]-N″-[4-[N-[2-[[(4-methylphenyl)phenyl-methyl]thio]ethyl]N-methylamino]butyl]guanidin

Die Herstellung erfolgt analog Beispiel 1 ausgehend von 0,87 g (2,5 mmol) N-Methyl-N-[2-[[(4-methylphenyl)phenyl-methyl]thio]ethyl]-1,4-butandiamin und 0,95 g (2,5 mmol) N-Cyano-N′-[2-[[(2-guanidino-4-thiazolyl)methyl]thio]ethyl]-O-phenyl-isoharnstoff. Nach chromatographischer Aufarbeitung analog Beispiel 1 erhält man die gereinigte Titelverbindung als zähes Öl; MS (+FAB-Methode): m/z ( rel. Int.[%]) = 624 ([M+H]+, 6), 181 (100); IR (KBr): 2161 $cm^{-1}$ (C≡N). Eine Probe wird zu analytischen Zwecken in das Salz der O,O′-Ditoluoylweinsäure überführt und aus Ether/Isopropanol umkristallisiert; Smp.: 134-140°C Zersetzung.

Beispiel 22: N-[3-[N-[3-(4-Fluorphenyl)-3-(2-pyridyl)propyl]-N-methylamino]propyl]-N′-[3-[3-(piperidinomethyl)phenoxy]propyl]-2-nitro-1,1-ethendiamin

Eine Mischung aus 0,74 g (2,5 mmol) N-[3-(4-Fluorphenyl)-3-(2-pyridyl)propyl]-N-methyl-1,3-propandiamin und einer äquimolaren Menge 1-Methylthio-1-[3-[3-(piperidinomethyl)phenoxy]propyl]amino-2-nitroethen werden in 20 ml Acetonitril 12 Stunden unter Rückfluss erhitzt. Danach wird der Ansatz vom Lösungsmittel befreit und die Titelverbindung mittels präparativer Dickschichtchromatographie (Eluens: Ethylacetat/ Methanol 9+1, Ammoniakatmosphäre) als zähes Öl isoliert; MS (+FAB-Methode): m/z ( rel. Int.[%]) = 619 ($[M+H]^+$ 2), 214 (100). Eine Probe wird zu analytischen Zwecken in das Salz der O,O′-Ditoluoylweinsäure überführt und aus Ether/Isopropanol umkristallisiert; Smp.: ab 102°C Zersetzung.

Beispiel 23: N-[7-[N-[3-(4-Chlorphenyl)-3-(2-pyridyl)propyl]-N-methylamino]heptyl]-N′-[2-[[(2-guanidino-4-thiazolyl)methyl]thio]ethyl]-2-nitro-1,1-ethendiamin

Die Herstellung erfolgt analog Beispiel 22 ausgehend von 0,83 g (1,7 mmol) 1-[7-[N-[3-(4-Chlorphenyl)-3-(2-pyridyl)propyl]-N-methylamino]heptyl]amino-1-methylthio-2-nitro-ethen und 0,5 g (2,1 mmol) 2-[[(2-Guanidino-4-thiazolyl)methyl]thio]-ethanamin. Nach chromatographischer Aufarbeitung analog Beispiel 1 erhält man die gereinigte Titelverbindung als Trockenschaum; MS (+FAB-Methode): m/z ( rel. Int.[%]) = 674 ($[M+H]^+$ 0,6), 230 (100).

Beispiel 24: N-[2-[N-[3-(4-Chlorphenyl)-3-(2-pyridyl)propyl]-N-methylamino]ethyl]-N′-[4-[3-(piperidinomethyl)phenoxy]butyl]harnstoff

Die Herstellung erfolgt analog Beispiel 63 ausgehend von 0,8 g (2,6 mmol) N-[3-(4-Chlorphenyl)-3-(2-pyridyl)propyl]-N-methyl-1,2-ethandiamin, einer äquimolaren Menge 1,1′-Carbonyldiimidazol und 0,69 g (2,7 mmol) 4-[3-(Piperidinomethyl)phenoxy]butanamin. Nach chromatographischer Aufarbeitung (Eluens: Methylenchlorid) analog Beispiel 63 erhält man die gereinigte Titelverbindung als Öl; MS (+FAB-Methode): m/z ( rel. Int.[%]) = 592 ([M+H]+, 2), 230 (100); IR (KBr): 1632 $cm^{-1}$ (C=O). Eine Probe wird zu analytischen Zwecken in das Salz der O,O′-Ditoluoylweinsäure überführt und aus Ether/Isopropanol umkristallisiert; Smp.: ab 124°C Zersetzung.

Beispiel 25: N-[5-[N-[3-(4-Chlorphenyl)-3-(2-pyridyl)propyl]-N-methylamino]pentyl]-N′-[3-[3-(piperidinomethyl)phenoxy]propyl]harnstoff

Die Herstellung erfolgt analog Beispiel 63 ausgehend von 1,29 g (3,6 mmol) N-[3-(4-Chlorphenyl)-3-(2-pyridyl)propyl]-N-methyl-1,5-pentandiamin, einer äquimolaren Menge 1,1′-Carbonyldiimidazol und 1,04 g (4,2 mmol) 3-[3-(Piperidinomethyl)phenoxy]propanamin. Nach chromatographischer Aufarbeitung (Eluens: Methylenchlorid) analog Beispiel 63 erhält man die gereinigte Titelverbindung als Öl; MS (+EI-80 eV): m/z ( rel. Int.[%]) = 619 ([M]+.,<1), 230 (17) 203 (100); IR (KBr): 1634 $cm^{-1}$ (C=O).

Beispiel 26: N-[4-[N-[3-(4-Chlorphenyl)-3-(2-pyridyl)propyl]-N-methylamino]butyl]-N′-[3-[3-(piperidinomethyl)phenoxy]propyl]harnstoff

Die Herstellung erfolgt analog Beispiel 63 ausgehend von 0,7 g (2,1 mmol) N-[3-(4-Chlorphenyl)-3-(2-pyridyl)propyl]-N-methyl-1,4-butandiamin, einer äquimolaren Menge 1,1′-Carbonyldiimidazol und 0,7 g (2,8 mmol) 3-[3-(Piperidinomethyl)phenoxy]propanamin. Nach chromatographischer Aufarbeitung (Eluens: Methylenchlorid/Methanol 95+5) analog Beispiel 63 erhält man die gereinigte Titelverbindung als Öl; MS (+FAB-Methode): m/z ( rel. Int.[%]) = 606 ($[M+H]^+$, 4), 230 (100); IR (KBr): 1643 $cm^{-1}$ (C=O).

Beispiel 27: N-[3-[N-[3-(4-Chlorphenyl)-3-(2-pyridyl)propyl]-N-methylamino]propyl]-N′-[3-[3-(piperidinomethyl)phenoxy]propyl]harnstoff

Die Herstellung erfolgt analog Beispiel 63 ausgehend von 0,7 g (2,2 mmol) N-[3-(4-Chlorphenyl)-3-(2-pyridyl)propyl]-N-methyl-1,3-propandiamin, einer äquimolaren Menge 1,1′-Carbonyldiimidazol und 0,55 g (2,2 mmol) 3-[3-(Piperidinomethyl)phenoxy]propanamin. Nach chromatographischer Aufarbeitung analog Beispiel 63 erhält man die gereinigte Titelverbindung als Öl. Eine Probe wird zu analytischen Zwecken in das Salz der Maleinsäure überführt und aus Ether/ Isopropanol umkristallisiert; Smp. (Ether/Ethanol): ab 65°C Zersetzung.

Beispiel 28: N-[2-[N-[3-(4-Chlorphenyl)-3-(2-pyridyl)propyl]-N-methylamino]ethyl]-N′-[3-[3-(piperidinomethyl)phenoxy]propyl]harnstoff

Die Herstellung erfolgt analog Beispiel 63 ausgehend von 0,92 g (3 mmol) N-[3-(4-Chlorphenyl)-3-(2-pyridyl)propyl]-N-methyl-1,2-ethandiamin, einer äquimolaren Menge 1,1′-Carbonyldiimidazol und 0,88 g (3,5 mmol) 3-[3-(Piperidinomethyl)phenoxy]propanamin. Nach chromatographischer Aufarbeitung (Eluens: Ethylacetat) analog Beispiel 63 erhält man die gereinigte Titelverbindung als Öl; MS (+FAB-Methode): m/z ( rel. Int.[%]) = 578 ($[M+H]^+$, 2), 230 (38), 78 (100); IR (KBr): 1637 $cm^{-1}$ (C=O). Eine Probe wird zu analytischen Zwecken in das Salz der O,O′-Ditoluoylweinsäure überführt und aus Ether/Isopropanol umkristallisiert; Smp.: 99-105°C.

Beispiel 29: N-[4-[N-[3-(4-Fluorphenyl)-3-(2-pyridyl)propyl]-N-methylamino]butyl]-N'-[3-[3-(piperidinomethyl)phenoxy]propyl]harnstoff

Die Herstellung erfolgt analog Beispiel 63 ausgehend von 0,7 g (2,2 mmol) N-[3-(4-Fluorphenyl)-3-(2-pyridyl)propyl]-N-methyl-1,4-butandiamin, einer äquimolaren Menge 1,1'-Carbonyldiimidazol und 0,6 g (2,4 mmol) 3-[3-(Piperidinomethyl)phenoxy]propanamin. Nach chromatographischer Aufarbeitung (Eluens: Ethylacetat/Methanol 95+5) analog Beispiel 63 erhält man die gereinigte Titelverbindung als Öl;MS (+FAB-Methode):m/z ( rel.Int.[%]) = 590 ($[M+H]^+$17), 214 (100);IR (KBr): 1631cm$^{-1}$ (C=O).

Beispiel 30: N-[3-[N-[3-(4-Fluorphenyl)-3-(2-pyridyl)propyl]-N-methylamino]propyl]-N'-[3-[3-(piperidinomethyl)phenoxy]propyl]harnstoff

Die Herstellung erfolgt analog Beispiel 63 ausgehend von 0,78 g (2,6 mmol) N-[3-(4-Fluorphenyl)-3-(2-pyridyl)propyl]-N-methyl-1,3-propandiamin, einer äquimolaren Menge 1,1'-Carbonyldiimidazol und 0,65 g (2,6 mmol) 3-[3-(Piperidinomethyl)phenoxy]propanamin. Nach chromatographischer Aufarbeitung (Eluens: Ethylacetat/Methanol 9+1) analog Beispiel 63 erhält man die gereinigte Titelverbindung als Öl; MS (+FAB-Methode): m/z ( rel. Int.[%]) = 576 ($[M+H]^+$, 7), 214 (100); IR (KBr): 1649 cm$^{-1}$ (C=O). Eine Probe wird zu analytischen Zwecken in das Salz der Maleinsäure überführt und aus Ether/Ethanol umkristallisiert; Smp. : 65-67°C.

Beispiel 31: N-[2-[N-[3-(4-Fluorphenyl)-3-(2-pyridyl)propyl]-N-methylamino]ethyl]-N'-[3-[3-(piperidinomethyl)phenoxy]propyl]harnstoff

Die Herstellung erfolgt analog Beispiel 63 ausgehend von 1g (3,5 mmol) N-[3-(4-Fluorphenyl)-3-(2-pyridyl)propyl]-N-methyl-1,2-ethandiamin, einer äquimolaren Menge 1,1'-Carbonyldiimidazol und 0,9 g (3,6 mmol) 3-[3-(Piperidinomethyl)phenoxy]propanamin. Nach chromatographischer Aufarbeitung (Eluens: Methylenchlorid) analog Beispiel 63 erhält man die gereinigte Titelverbindung als Öl; MS (EI-80 eV): m/z ( rel. Int.[%]) = 561 ($[M]^{+.}$, 3), 84 (70), 214 (100). Eine Probe wird zu analytischen Zwecken in das Hydrochlorid überführt und aus Ether/ Ethanol umkristallisiert; Smp.: 111-115°C.

Beispiel 32: N-[4-[N-[3-Phenyl-3-(2-pyridyl)propyl]-N-methylamino]butyl]-N'-[3-[3-(piperidinomethyl)phenoxy]propyl]harnstoff

Die Herstellung erfolgt analog Beispiel 63 ausgehend von 0,7 g (2,3 mmol) N-Methyl-N-[3-phenyl-3-(2-pyridyl)propyl]-1,4-butandiamin, einer äquimolaren Menge 1,1'-Carbonyldiimidazol und 0,7 g (2,8 mmol) 3-[3-(Piperidinomethyl)phenoxy]propanamin. Nach chromatographischer Aufarbeitung analog Beispiel 63 erhält man die gereinigte Titelverbindung als Öl; MS (EI-80 eV): m/z ( rel. Int.[%]) = 571 ($[M]^{+.}$, 4), 84 (22), 169 (100).

Beispiel 33: N-[3-[N-[3-Phenyl-3-(2-pyridyl)propyl]-N-methylamino]propyl]-N'-[3-[3-(piperidinomethyl) phenoxy]propyl]harnstoff

Die Herstellung erfolgt analog Beispiel 63 ausgehend von 0,74 g (2,6 mmol) N-Methyl-N-[3-phenyl-3-(2-pyridyl)propyl]-1,3-propandiamin, einer äquimolaren Menge 1,1'-Carbonyldiimidazol und 0,65 g (2,6 mmol) 3-[3-(Piperidinomethyl)phenoxy]propanamin. Nach chromatographischer Aufarbeitung (Eluens: Ethylacetat/ Methanol 9+1) analog Beispiel 63 erhält man die gereinigte Titelverbindung als Öl; MS (+FAB-Methode): m/z ( rel. Int.[%]) = 558 ($[M+H]^{\circ}$, 11), 196 (100); IR (KBr): 1639 cm$^{-1}$ (C=O). Eine Probe wird zu analytischen Zwecken in das Salz der Maleinsäure überführt und aus Ether/ Ethanol umkristallisiert; Smp.: ab 70°C Zersetzung.

Beispiel 34: N-[2-[N-[3-Phenyl-3-(2-pyridyl)propyl]-N-methylamino]ethyl]-N'-[3-[3-(piperidinomethyl)phenoxy] propyl]harnstoff

Die Herstellung erfolgt analog Beispiel 63 ausgehend von 0,33 g (1,2 mmol) N-Methyl-N-[3-phenyl-3-(2-pyridyl)propyl]-1,2-ethandiamin, einer äquimolaren Menge 1,1'-Carbonyldiimidazol und 0,4 g (1,6 mmol) 3-[3-(Piperidinomethyl)phenoxy]propanamin in 10 ml absolutem Tetrahydrofuran. Nach chromatographischer Aufarbeitung (Eluens: Ethylacetat) analog Beispiel 63 erhält man die gereinigte Titelverbindung als Öl; MS (EI-80 eV): m/z ( rel. Int.[%]) = 543 ($[M]^{+.}$; 1), 84 (100). Eine Probe wird zu analytischen Zwecken in das Hydrochlorid überführt und aus Ether/Ethanol umkristallisiert; Smp.: ab 130°C Zersetzung.

Beispiel 35: N-[8-[N-[3-(4-Chlorphenyl)-3-(2-pyridyl)propyl]-N-methylamino]octyl]-N'-[2-[[(2-guanidino-4-thiazolyl)methyl]thio]ethyl]harnstoff

Die Herstellung erfolgt analog Beispiel 63 ausgehend von 0,6 g (1,6 mmol) N-[3-(4-Chlorphenyl)-3-(2-pyridyl)propyl]-N-methyl-1,8-octandiamin, einer äquimolaren Menge 1,1'-Carbonyldiimidazol und 0,4 g (1,7 mmol) 2-[[(2-Guanidino-4-thiazolyl)methyl]thio]ethanamin. Nach chromatographischer Aufarbeitung (Eluens: Chloroform/Methanol 9+1) analog Beispiel 63 erhält man die gereinigte Titelverbindung als Trockenschaum; MS (+FAB-Methode, DMSO/MNBA): m/z ( rel. Int.[%]) = 645 ($[M+H]^+$,<1), 234 (100); IR (KBr): 1638 $cm^{-1}$ (C=O). Eine Probe wird zu analytischen Zwecken in das Salz der O,O'-Ditoluoylweinsäure überführt und aus Ether/Isopropanol umkristallisiert; Smp.: 135-140°C.

Beispiel 36: N-[7-[N-[3-(4-Chlorphenyl)-3-(2-pyridyl)propyl]-N-methylamino]heptyl]-N'-[2-[[(2-guanidino-4-thiazolyl)methyl]thio]ethyl]harnstoff

Die Herstellung erfolgt analog Beispiel 63 ausgehend von 0,73 g (1,9 mmol) N-[3-(4-Chlorphenyl)-3-(2-pyridyl)propyl]-N-methyl-1,7-heptandiamin, einer äquimolaren Menge 1,1'-Carbonyldiimidazol und 0,45 g (2 mmol) 2-[[(2-Guanidino-4-thiazolyl)methyl]thio]ethanamin [DE-A-2817078, ICI; C.A. 90, 87452d (1979)]. Nach chromatographischer Aufarbeitung (Eluens: Methylenchlorid/Methanol 9+1) analog Beispiel 63 erhält man die gereinigte Titelverbindung als Trockenschaum; MS (+FAB-Methode): m/z ( rel. Int. [%]) = 631 ($[M+H]^+$, 3), 230 (31), 154 (100); IR (KBr): 1605 $cm^{-1}$ (C=O). Eine Probe wird zu analytischen Zwecken in das Salz der O,O'-Ditoluoylweinsäure überführt und aus Ether/Isopropanol umkristallisiert; Smp.: 132-134°C Zersetzung.

Die Ausgangsverbindung wird wie folgt hergestellt:

Eine Lösung von 3-(4-Chlorphenyl)-3-(2-pyridyl)propanamin wird wie unter Beispiel 1 beschrieben zu 3-(4-Chlorphenyl)-3-(2-pyridyl)-N-methyl-1-propanamin [A. Buschauer, J. Med. Chem. 32, 1963 (1989)] umgesetzt und nach säulenchromatographischer Reinigung (Ethylacetat/ammoniak. Methanol 99+1) in einer Ansatzgrösse von 10-30 mmol zusammen mit einer äquivalenten Menge 7-Bromheptanonitril und einem doppelt molaren Überschuss an $Na_2CO_3$ in 20 ml Acetonitril 2 Stunden bei 60°C gerührt. Nach Ende der Reaktion (dc-Kontrolle) wird der Ansatz mit 20 ml Wasser versetzt und mehrmals mit Toluol extrahiert. Die vereinigten organischen Extrakte werden mit Wasser gewaschen, über $Na_2SO_4$ getrocknet und mit einer kleinen Menge Kieselgel für die SC entfärbt. Nach Eindampfen im Vakuum erhält man das Reaktionsprodukt, 7-[N-[3-(4-Chlorphenyl)-3-(2-pyridyl)propyl]-N-methylamino] -heptannitril in genübender Reinheit. Das so dargestellte Nitril wird in Ether gelöst und in eine mit Eis gekühlte und gerührte Suspension von $LiAlH_4$ (1,5 Äquivaltente) in absolutem Ether (Gesamtvolumen ca. 50 ml) getropft und 2 Stunden bei Raumtemperatur belassen. Nach vollständiger Umsetzung des Eduktes wird der Ansatz mit wassergesättigtem Ether und 2-3 ml 10 %iger NaOH versetzt und über Nacht bei Raumtemperatur gerührt. Nach Absaugen der Anorganica wird das Filtrat im Vakuum eingedampft und das resultierende N-[3-(4-Chlorphenyl)-3-(2-pyridyl)propyl]-N-methyl-1,7-heptandiamin bei genügender Reinheit weiter umgesetzt oder mittels präparativer Dickschichtchromatographie isoliert.

Beispiel 37: N-[6-[N-[3-(4-Chlorphenyl)-3-(2-pyridyl)propyl]-N-methylamino]hexyl]-N'-[2-[[(2-guanidino-4-thiazolyl)methyl]thio]ethyl]harnstoff

Die Herstellung erfolgt analog Beispiel 63 ausgehend von 0,9 (2,5 mmol) N-[3-(4-Chlorphenyl)-3-(2-pyridyl)propyl]-N-methyl-1,6-hexandiamin, einer äquimolaren Menge 1,1'-Carbonyldiimidazol und 0,6 g (2,6 mmol) 2-[[(2-Guanidino-4-thiazolyl)methyl]thio]ethanamin. Nach chromatographischer Aufarbeitung (Eluens: Methylenchlorid) analog Beispiel 63 erhält man die gereinigte Titelverbindung als Öl; MS (+FAB-Methode): m/z ( rel. Int.[%]) = 617 ($[M+H]^+$,6), 230 (100), 155 (28); IR (KBr): 1641 $cm^{-1}$ (C=O).

Beispiel 38: N-[5-[N-[3-(4-Chlorphenyl)-3-(2-pyridyl)propyl]-N-methylamino]pentyl]-N'-[2-[[(2-guanidino-4-thiazolyl)methyl]thio]ethyl]harnstoff

Die Herstellung erfolgt analog Beispiel 63 ausgehend von 1,04 g (3 mmol) N-[3-(4-Chlorphenyl)-3-(2-pyridyl)propyl]-N-methyl-1,5-pentandiamin, einer äquimolaren Menge 1,1'-Carbonyldiimidazol und 0,75 g (3,3 mmol) 2-[[(2-Guanidino-4-thiazolyl)methyl]thio]ethanamin. Nach chromatographischer Aufarbeitung (Eluens: Chloroform/Methanol 9+1) analog Beispiel 63 erhält man die gereinigte Titelverbindung als Trockenschaum; MS (+FAB-Methode): m/z ( rel. Int.[%]) = 603 ($[M+H]^+$, 1), 155 (18), 230 (100); IR (KBr): 1640 $cm^{-1}$ (C=O).

Beispiel 39: N-[4-[N-[3-(4-Chlorphenyl)-3-(2-pyridyl)propyl]-N-methylamino]butyl]-N′-[2-[[(2-guanidino-4-thiazolyl)methyl]thio]ethyl]harnstoff

Die Herstellung erfolgt analog Beispiel 63 ausgehend von 0,75 g (2 mmol) N-[3-(4-Chlorphenyl)-3-(2-pyridyl)propyl]-N-methyl-1,4-butandiamin, einer äquimolaren Menge 1,1′-Carbonyldiimidazol und 0,54 g (2,3 mmol) 2-[[(2-Guanidino-4-thiazolyl)methyl]thio]ethanamin. Nach chromatographischer Aufarbeitung (Eluens: Methylenchlorid/Methanol 9+1) analog Beispiel 63 erhält man die gereinigte Titelverbindung als Trockenschaum; MS (+FAB-Methode): m/z (rel. Int.[%]) = 589 ($[M+H]^+$, 2), 230 (100); IR (KBr): 1650 $cm^{-1}$ (C=O). Eine Probe wird zu analytischen Zwecken in das Salz der Maleinsäure überführt und aus Ether/Ethanol umkristallisiert; Smp.: ab 90°C Zersetzung.

Beispiel 40: N-[3-[N-[3-(4-Chlorphenyl)-3-(2-pyridyl)propyl]-N-methylamino]propyl]-N′-[2-[[(2-guanidino-4-thiazolyl)methyl]thio]ethyl]harnstoff

Die Herstellung erfolgt analog Beispiel 63 ausgehend von 0,9 g (2,8 mmol) N-[3-(4-Chlorphenyl)-3-(2-pyridyl)propyl]-N-methyl-1,3-propandiamin, einer äquimolaren Menge 1,1′-Carbonyldiimidazol und 0,73 g (3,1 mmol) 2-[[(2-Guanidino-4-thiazolyl)methyl]thio]ethanamin. Nach chromatographischer Aufarbeitung (Eluens: Ethylacetat/Methanol 85+15) analog Beispiel 63 erhält man die gereinigte Titelverbindung als Trockenschaum; MS (+FAB-Methode): m/z (rel. Int.[%]) = 575 ($[M+H]^+$, 3), 155 (22), 230 (100); IR (KBr): 1646 $cm^{-1}$ (C=O).

Beispiel 41: N-[2-[N-[3-(4-Chlorphenyl)-3-(2-pyridyl)propyl]-N-methylamino]ethyl]-N′-[2-[[(2-guanidino-4-thiazolyl)methyl]thio]ethyl]harnstoff

Die Herstellung erfolgt analog Beispiel 63 ausgehend von 0,9 g (2,9 mmol) N-[3-(4-Chlorphenyl)-3-(2-pyridyl)propyl]-N-methyl-1,2-ethandiamin, einer äquimolaren Menge 1,1′-Carbonyldiimidazol und 0,75 g (3,2 mmol) 2-[[(2-Guanidino-4-thiazolyl)methyl]thio]ethanamin. Nach chromatographischer Aufarbeitung (Eluens: Methylenchlorid/Methanol 95+5) analog Beispiel 63 erhält man die gereinigte Titelverbindung als Öl; MS (+FAB-Methode): m/z ( rel. Int.[%]) = 561 ($[M+H]^+$, 3), 155 (26), 230 (100); IR (KBr): 1650 $cm^{-1}$ (C=O). Eine Probe wird zu analytischen Zwecken in das Salz der O,O′-Ditoluoylweinsäure überführt und aus Ether/Isopropanol umkristallisiert; Smp.: ab 140°C Zersetzung.

Beispiel 42: N-[7-[N-[3-(4-Fluorphenyl)-3-(2-pyridyl)propyl]-N-methylamino]heptyl]-N′-[2-[[(2-guanidino-4-thiazolyl)methyl]thio]ethyl]harnstoff

Die Herstellung erfolgt analog Beispiel 63 ausgehend von 0,9 g (2,5 mmol) N-[3-(4-Fluorphenyl)-3-(2-pyridyl)propyl]-N-methyl-1,7-heptandiamin, einer äquimolaren Menge 1,1′-Carbonyldiimidazol und 0,65 g (2,8 mmol) 2-[[(2-Guanidino-4-thiazolyl)methyl]thio]ethanamin. Nach chromatographischer Aufarbeitung (Eluens: Methylenchlorid) analog Beispiel 63 erhält man die gereinigte Titelverbindung als Trockenschaum; MS (+FAB-Methode): m/z (rel. Int.[%]) = 615 ($[M+H]^+$ 2), 214 (100); IR (KBr): 1632 $cm^{-1}$ (C=O).

Beispiel 43: N-[4-[N-[3-(4-Fluorphenyl)-3-(2-pyridyl)propyl]-N-methylamino]butyl]-N′-[2-[[(2-guanidino-4-thiazolyl)methyl]thio]ethyl]harnstoff

Die Herstellung erfolgt analog Beispiel 63 ausgehend von 0,95 g (3,5 mmol) N-[3-(4-Fluorphenyl)-3-(2-pyridyl)propyl]-N-methyl-1,4-butandiamin, einer äquimolaren Menge 1,1′-Carbonyldiimidazol und 0,82 g (3,5 mmol) 2-[[(2-Guanidino-4-thiazolyl)methyl]thio]ethanamin. Nach chromatographischer Aufarbeitung (Eluens: Ethylacetat/Methanol 9+1) analog Beispiel 63 erhält man die gereinigte Titelverbindung als Trockenschaum; MS (+FAB-Methode): m/z (rel. Int.[%]) = 573 ($[M+H]^+$ 2), 214 (100); IR (KBr): 1632 $cm^{-1}$ (C=O). Eine Probe wird zu analytischen Zwecken in das Salz der O,O′-Ditoluoylweinsäure überführt und aus Ether/ Isopropanol umkristallisiert; Smp.: ab 120°C Zersetzung.

Beispiel 44: N-[2-[[(2-Guanidino-4-thiazolyl)methyl]thio]ethyl]-N′-[7-[N-[3-phenyl-3-(2-pyridyl)propyl]-N-methylamino]heptyl]harnstoff

Die Herstellung erfolgt analog Beispiel 63 ausgehend von 0,85 g (2,5 mmol) N-Methyl-N-[3-phenyl-3-(2-pyridyl)propyl]-1,7-heptandiamin, einer äquimolaren Menge 1,1′-Carbonyldiimidazol und 0,65 g (2,8 mmol) 2-[[(2-Guanidino-4-thiazolyl)methyl]thio]ethanamin. Nach chromatographischer Aufarbeitung (Eluens: Methylenchlorid) analog Beispiel 63 erhält man die gereinigte Titelverbindung als Öl; MS (+FAB-Methode): m/z (rel.

Int.[%]) = 597 ([M+H]$^+$, 7), 196 (100); IR (KBr): 1655 $cm^{-1}$ (C=O).

Beispiel 45: N-[2-[[(2-Guanidino-4-thiazolyl)methyl]thio]ethyl]-N′-[4-[N-[3-phenyl-3-(2-pyridyl)propyl]-N-methylamino]butyl]harnstoff

Die Herstellung erfolgt analog Beispiel 63 ausgehend von 0,89 g (3 mmol) N-Methyl-N-[3-phenyl-3-(2-pyridyl)propyl]-1,4-butandiamin, einer äquimolaren Menge 1,1′-Carbonyldiimidazol und 0,75 g (3,2 mmol) 2-[[(2-Guanidino-4-thiazolyl)methyl]thio]ethanamin. Nach chromatographischer Aufarbeitung (Eluens: Chloroform/Methanol 9+1) analog Beispiel 63 erhält man die gereinigte Titelverbindung als Trockenschaum; MS (+FAB-Methode): m/z (rel. Int.[%]) = 555 ([M+H]$^+$ 1), 196 (100); IR (KBr): 1610 $cm^{-1}$ (C=O). Eine Probe wird zu analytischen Zwecken in das Salz der Maleinsäure überführt und aus Ether/ Ethanol umkristallisiert; Smp.: ab 80°C Zersetzung.

Beispiel 46: N-[2-[[(2-Guanidino-4-thiazolyl)methyl]thio]ethyl]-N′-[3-[N-[3-phenyl-3-(2-pyridyl)propyl]-N-methylamino]propyl]harnstoff

Die Herstellung erfolgt analog Beispiel 63 ausgehend von 0,85 g (3 mmol) N-Methyl-N-[3-phenyl-3-(2-pyridyl)propyl]-1,3-propandiamin, einer äquimolaren Menge 1,1′-Carbonyldiimidazol und 0,75 g (3,2 mmol) 2-[[(2-Guanidino-4-thiazolyl)methyl]thio]ethanamin. Nach chromatographischer Aufarbeitung (Eluens: Chloroform/Methanol 9+1) analog Beispiel 63 erhält man die gereinigte Titelverbindung als Öl; MS (+FAB-Methode): m/z ( rel. Int.[%]) = 541 ([M+H]$^+$, 2), 196 (100); IR (KBr): 1650 $cm^{-1}$ (C=O). Eine Probe wird zu analytischen Zwecken in das Salz der Maleinsäure überführt und aus Ether/Ethanol umkristallisiert; Smp.: 95-98°C.

Beispiel 47: N-[2-[[(2-Guanidino-4-thiazolyl)methyl]thio]ethyl]-N′-[2-[N-[3-phenyl-3-(2-pyridyl)propyl]-N-methylamino]ethyl]harnstoff

Die Herstellung erfolgt analog Beispiel 63 ausgehend von 1g (3,7 mmol) N-Methyl-N-[3-phenyl-3-(2-pyridyl)propyl]-1,2-ethandiamin, einer äquimolaren Menge 1,1′-Carbonyldiimidazol und 1,15 g (5 mmol) 2-[[(2-Guanidino-4-thiazolyl)methyl]thio]ethanamin. Nach chromatographischer Aufarbeitung (Eluens: Ethylacetat/ Methanol 9+1) analog Beispiel 63 erhält man die gereinigte Titelverbindung als Trockenschaum; MS (+FAB-Methode): m/z ( rel. Int.[%]) = 527 ([M+H]$^+$ 3), 196 (100).

Beispiel 48: N-[7-[N-[3-(4-Chlorphenyl)-3-(2-pyridyl)propyl]-N-methylamino]heptyl]-N′-[2-[[(2-guanidino-4-thiazolyl)methyl]thio]ethyl]thioharnstoff

Die Herstellung erfolgt analog Beispiel 99 ausgehend von 0,84 g (2,2 mmol) N-[3-(4-Chlorphenyl)-3-(2-pyridyl)propyl]-N-methyl-1,7-heptandiamin und 0,56 g (2,4 mmol) 2-[[(2-Guanidino-4-thiazolyl)methyl]thio]ethanamin. Nach chromatographischer Aufarbeitung (Eluens: Chloroform/Methanol 9+1) analog Beispiel 99 erhält man die gereinigte Titelverbindung als Trockenschaum; MS (+FAB-Methode): m/z ( rel. Int. [%]) = 647 ([M+H]$^+$, 7), 230 (100).

Beispiel 49: N-[4-[N-[3-(4-Chlorphenyl)-3-(2-pyridyl)propyl]-N-methylamino]butyl]-N′-[2-[[5-methylimidazol-4-yl)methyl]thio]ethyl]harnstoff

Die Herstellung erfolgt analog Beispiel 63 ausgehend von 0,8 g (2,4 mmol) N-[3-(4-Chlorphenyl)-3-(2-pyridyl)propyl]-N-methyl-1,4-butandiamin, einer äquimolaren Menge 1,1′-Carbonyldiimidazol und 0,5 g (2,9 mmol) 2-[[(5-Methylimidazol-4-yl)methyl]thio]ethanamin [R.W. Brimblecombe et al., J. Int. Med. Res. 3, 86 (1975)]. Nach chromatographischer Aufarbeitung (Eluens: Ethylacetat/Methanol 9+1) analog Beispiel 63 erhält man die gereinigte Titelverbindung als kristallinen Feststoff, der aus Ether umkristallisiert wird; Smp.: 97-98°C; MS (+FAB-Methode): m/z (rel. Int.[%]) = 529 ([M+H]$^+$, 10), 230 (100); IR (KBr): 1643 $cm^{-1}$ (C=O).

Beispiel 50: N-[3-[N-[3-(4-Chlorphenyl)-3-(2-pyridyl)propyl]-N-methylamino]propyl]-N′-[2-[[(5-methylimidazol-4-yl)methyl]thio]ethyl]harnstoff

Die Herstellung erfolgt analog Beispiel 63 ausgehend von 0,8 g (2,5 mmol) N-[3-(4-Chlorphenyl)-3-(2-pyridyl)propyl]-N-methyl-1,3-propandiamin, einer äquimolaren Menge 1,1′-Carbonyldiimidazol und 0,5 g (2,9 mmol) 2-[[(5-Methylimidazol-4-yl)methyl]thio]ethanamin. Nach chromatographischer Aufarbeitung (Eluens: Ethylacetat/Methanol 9+1) analog Beispiel 63 erhält man die gereinigte Titelverbindung als kristallinen Feststoff;

Smp.(Ether): 102-104°C; MS (+FAB-Methode): m/z ( rel. Int.[%]) = 515 ($[M+H]^+$, 10), 95 (75), 230 (100).

Beispiel 51: N-[2-[N-[3-(4-Chlorphenyl)-3-(2-pyridyl)propyl]-N-methylamino]ethyl]-N′-[2-[[(5-methylimidazol-4-yl)methyl]thio]ethyl]harnstoff

Die Herstellung erfolgt analog Beispiel 63 ausgehend von 0,8 g (2,6 mmol) N-[3-(4-Chlorphenyl)-3-(2-pyridyl)propyl]-N-methyl-1,2-ethandiamin, einer äquimolaren Menge 1,1′-Carbonyldiimidazol und 0,5, g (2,8 mmol) 2-[[(5-Methylimidazol-4-yl)methyl]thio]ethanamin. Nach chromatographischer Aufarbeitung (Eluens: Ethylacetat/Methanol 9+1) analog Beispiel 63 erhält man die gereinigte Titelverbindung als kristallinen Feststoff; Smp.(Ether): 114°C; MS (+FAB-Methode): m/z ( rel. Int.[%]) = 501 ($[M+H]^+$, 15), 95 (63), 230 (100); IR (KBr): 1640 $cm^{-1}$ (C=O).

Beispiel 52: N-Cyano-N′-[2-[N-[2-[N-(4-methoxybenzyl)-N-(2-pyridyl)amino]ethyl]-N-methylamino]ethyl]-N″-[3-[3-(piperidinomethyl)phenoxy]propyl]guanidin

Die Herstellung erfolgt analog Beispiel 1 ausgehend von 0,53 g (1,7 mmol) N-[2-[N-(4-Methoxybenzyl)-N-(2-pyridyl)amino]ethyl]-N-methyl-1,2-ethandiamin und der äquimolaren Menge N-Cyano-O-phenyl-N′-[3-[3-(piperidinomethyl)phenoxy]propyl]isoharnstoff. Nach chromatographischer Aufarbeitung analog Beispiel 1 erhält man die gereinigte Titelverbindung als zähes Öl, das aus absolutem Ether bei -20°C auskristallisiert; MS (EI 70 eV): m/z (rel. Int. [%]) = 612 ($[M]^+$. 1); IR (KBr): 2164 $cm^{-1}$ (C≡N); Smp.: 88°C (Ether).

Die Ausgangsverbindung wird wie folgt hergestellt:

Vom 2-[N-(2-Aminoethyl)-N-(4-methoxybenzyl)-amino]pyridin [USP 4 532 246 (20.12.1983); C.A. 102, 6208v (1984)] werden 11,23 g (43,64 mmol) in Ether gelöst und mit 10 %iger Natronlauge unterschichtet. Unter Rühren und Kühlen mit Eiswasser werden 4,73 g (43,64 mmol) Chlorameisensäureethylester zu dem 2-Phasensystem tropfenweise so zugegeben, dass sich die dabei entstehende Trübung zunächst auflösen muss, bevor weiterer Ester zugetropft werden kann. Nach vollständiger Umsetzung werden die Phasen voneinander getrennt, die etherische Phase mit $Na_2SO_4$ getrocknet und der Ether im schwachen Vakuum abdestilliert. Man erhält den N-(4-Methoxybenzyl)-N-(2-pyridyl)-2-aminoethanaminsäureethylester als farbloses Öl. Dieses wird, gelöst in 20 ml absolutem THF, unter Eiskühlung und Rühren langsam in eine Suspension von 2,28 g (60 mmol) $LiAlH_4$ in 100 ml absolutem Ether eingetragen. Nach 30 min Rühren bei Raumtemperatur wird der Reaktionsansatz noch 1 Stunde unter Rückfluss erhitzt. Nach Abkühlen wird der Ansatz unter Eiskühlung mit wassergesättigtem Ether und tropfenweiser Zugabe von wenig 10 %iger Natronlauge hydrolysiert, bis die exotherme Reaktion beendet ist und sich ein weisser Niederschlag bildet. Man lässt weitere 30 min rühren, saugt dann ab und wäscht den Niederschlag mehrmals mit Ether nach.

Das Filtrat wird mit Wasser gewaschen, über $Na_2SO_4$ getrocknet und im Vakuum bis zur Trockne eingeengt. Man erhält das N-(4-Methoxybenzyl)-N-(2-pyridyl)-N′-methyl-1,2-ethandiamin. Dieses wird anschliessend mit der äquimolaren Menge Chloracetonitril, der dreifach molaren Menge $Na_2CO_3$ und einer Spatelspitze KI in 60 ml wasserfreiem DMF für 2 Stunden auf 80°C erhitzt. Nach Abkühlung auf Raumtemperatur wird soviel Wasser zum Reaktionsansatz gegeben bis sich der Niederschlag gelöst hat. Der Ansatz wird mit Toluol extrahiert. Die vereinigten organischen Phasen werden mit $MgSO_4$ getrocknet und das Toluol im Vakuum abdestilliert. Das resultierende bräunliche Öl wird in 20 ml absolutem THF gelöst und unter Eiskühlung und Rühren langsam in eine doppeltmolare Suspenison von $LiAlH_4$ in 50 ml absolutem Ether eingetragen. Nach 30 min Rühren wird der Ansatz unter Eiskühlung mit wassergesättigtem Ether und tropfenweiser Zugabe von wenig 10 %iger Natronlauge hydrolysiert bis die exotherme Reaktion beendet ist und sich ein weisser Niederschlag bildet. Man lässt weitere 30 min Rühren, saugt dann ab und wäscht den Niederschlag mehrmals mit Ether nach. Das Filtrat wird mit Wasser gewaschen, über $Na_2SO_4$ getrocknet und im Vakuum bis zur Trockne eingeengt. Man erhält N-[2-[N-(4-Methoxybenzyl)-N-(2-pyridyl)amino]ethyl]-N-methyl -1,2-ethandiamin in genügender Reinheit für die nachfolgende Reaktion.

Beispiel 53: N-Cyano-N′-[3-[N-[2-[N-(4-methoxybenzyl)-N-(2-pyridyl)amino]ethyl]-N-methylamino]propyl]-N″-[3-[3-(piperidinomethyl)phenoxy]propyl]guanidin

Die Herstellung erfolgt analog Beispiel 1 ausgehend von 0,52 g (1,6 mmol) N-[2-[N-(4-Methoxybenzyl)-N-(2-pyridyl)amino]ethyl]-N-methyl-1,3-propandiamin und der äquimolaren Menge N-Cyano-O-phenyl-N′-[3-[3-(piperidinomethyl)phenoxy]propyl]isoharnstoff. Nach chromatographischer Aufarbeitung analog Beispiel 1 erhält man die gereinigte Titelverbindung als zähes Öl, das aus absolutem Ether bei -20°C auskristallisiert; MS (+FAB-Methode): m/z (rel. Int. [%]) = 627 ($[M+H]^+$, 6), 241 (11), 121 (100); IR (KBr): 2165 $cm^{-1}$ (C≡N); Smp.: 62-65°C (Ether).

Beispiel 54: N-Cyano-N′-[4-[N-[2-[N-(4-methoxybenzyl)-N-(2-pyridyl)amino]ethyl]-N-methylamino]butyl]-N″-[3-[3-(piperidinomethyl)phenoxy]propyl]guanidin

Die Herstellung erfolgt analog Beispiel 1 ausgehend von 0,53 g (1,5 mmol) N-[2-[N-(4-Methoxybenzyl)-N-(2-pyridyl)amino]ethyl]N-methyl-1,4-butandiamin und der äquimolaren Menge N-Cyano-O-phenyl-N′-[3-[3-(piperidinomethyl)phenoxy]propyl]isoharnstoff. Nach chromatographischer Aufarbeitung analog Beispiel 1 erhält man die gereinigte Titelverbindung als zähes Öl. Eine Probe wird zu analytischen Zwecken in das Salz der Weinsäure überführt und aus Ether/Ethanol/Isopropanol umkristallisiert; MS (+FAB-Methode): m/z (rel. Int. [%]) = 641 ($[M+H]^+$, 1), 241 (7), 121 (100); IR (KBr): 2166 $cm^{-1}$ (C≡N); Smp.: 106-108°C (Ether/Ethanol/Isopropanol).

Beispiel 55: N-Cyano-N′-[6-[N-[2-[N-(4-methoxybenzyl)-N-(2-pyridyl)amino]ethyl]-N-methylamino]hexyl]-N″-[3-[3-(piperidinomethyl)phenoxy]propyl]guanidin

Die Herstellung erfolgt analog Beispiel 1 ausgehend von 0,56 g (1,52 mmol) N-[2-[N-(4-Methoxybenzyl)-N-(2-pyridyl)amino]ethyl]-N-methyl-1,6-hexandiamin und der äquimolaren Menge N-Cyano-O-phenyl-N′-[3-[3-(piperidinomethyl)phenoxy]propyl]isoharnstoff. Nach chromatographischer Aufarbeitung analog Beispiel 1 erhält man die gereinigte Titelverbindung als zähes Öl; MS (+FAB-Methode): m/z (rel. Int. [%]) = 669 ($[M+H]^+$, 5), 121 (100); IR (KBr): 2163 $cm^{-1}$ (C≡N). Eine Probe wird zu analytischen Zwecken in das Salz der Weinsäure in einem Lösungsmittelgemisch Ether/Ethanol überführt; Smp.: 79°C (Ether/Ethanol).

Beispiel 56: N-Cyano-N′-[2-[N-[2-[N-(4-fluorbenzyl)-N-(2-pyridyl)amino]ethyl]-N-methylamino]ethyl]-N″-[3-[3-(piperidinomethyl)phenoxy]propyl]guanidin

Die Herstellung erfolgt analog Beispiel 1 ausgehend von 0,30 g (1,0 mmol) N-[2-[N-(4-Fluorbenzyl)-N-(2-pyridyl)amino]ethyl]-N-methyl-1,2-ethandiamin und der äquimolaren Menge N-Cyano-O-phenyl-N′-[3-[3-(piperidinomethyl)phenoxy]propyl]isoharnstoff. Nach chromatographischer Aufarbeitung analog Beispiel 1 erhält man die gereinigte Titelverbindung als zähes Öl; MS (+FAB-Methode): m/z (rel. Int. [%]) = 601 ($[M+H]^+$, 7), 76 (100); IR (KBr): 2169 $cm^{-1}$ (C≡N). Zur weiteren Analytik wird ein Teil der Substanz in das Salz der Weinsäure überführt und aus Ethanol/Petrolether umkristallisiert; Smp.: 106°C (Ethanol/Petrolether).

Beispiel 57: N-Cyano-N′-[3-[N-[2-[N-(4-fluorbenzyl)-N-(2-pyridyl)amino]ethyl]-N-methylamino]propyl]-N″-[3-[3-(piperidinomethyl)phenoxy]propyl]guanidin

Die Herstellung erfolgt analog Beispiel 1 ausgehend von 0,63 g (2,0 mmol) N-[2-[N-(4-Fluorbenzyl)-N-(2-pyridyl)amino]ethyl]-N-methyl-1,3-propandiamin und der äquimolaren Menge N-Cyano-O-phenyl-N′-[3-[3-(piperidinomethyl)phenoxy]propyl]isoharnstoff. Nach chromatographischer Aufarbeitung analog Beispiel 1 erhält man die gereinigte Titelverbindung als zähes Öl; MS (+FAB-Methode): m/z (rel. Int. [%]) = 615 ($[M+H]^+$, 14), 229 (100), 109 (96); IR (KBr): 2167 $cm^{-1}$ (C≡N). Zur weiteren Analytik wird ein Teil der Substanz in das Salz der Weinsäure in einem Lösungsmittelgemisch Ethanol/Ether überführt; Smp.: 65-68°C (Ethanol/Ether).

Beispiel 58: N-Cyano-N′-[4-[N-[2-[N-(4-fluorbenzyl)-N-(2-pyridyl)amino]ethyl]-N-methylamino]butyl]-N″-[3-[3-(piperidinomethyl)phenoxy]propyl]guanidin

Die Herstellung erfolgt analog Beispiel 1 ausgehend von 0,33 g (1,0 mmol) N-[2-[N-(4-Fluorbenzyl)-N-(2-pyridyl)amino]ethyl]-N-methyl-1,4-butandianmin und der äquimolaren Menge N-Cyano-O-phenyl-N′-[3-[3-(piperidinomethyl)phenoxy]propyl]isoharnstoff. Nach chromatographischer Aufarbeitung analog Beispiel 1 erhält man die gereinigte Titelverbindung als zähes Öl; MS (+FAB-Methode): m/z (rel. Int. [%]) = 629 ($[M+H]^+$, 5), 229 (84), 109 (100); IR (KBr): 2166 $cm^{-1}$ (C≡N). Zur weiteren Analytik wird ein Teil der Substanz in das Salz der Weinsäure überführt und aus Ethanol/Acetonitril/Isopropanol umkristallisiert; Smp.: 84-86°C (Ethanol/Acetonitril/Isopropanol).

Beispiel 59: N-Cyano-N′-[5-[N-[2-[N-(4-fluorbenzyl)-N-(2-pyridyl)amino]ethyl]-N-methylamino]pentyl]-N″-[3-[3-(piperidinomethyl)phenoxy]propyl]guanidin

Die Herstellung erfolgt analog Beispiel 1 ausgehend von 0,54 g (1,6 mmol) N-[2-[N-(4-Fluorbenzyl)-N-(2-pyridyl)amino]ethyl]-N-methyl-1,5-pentandiamin und der äquimolaren Menge N-Cyano-O-phenyl-N′-[3-[3-(piperidinomethyl)phenoxy]propyl]isoharnstoff. Nach chromatographischer Aufarbeitung analog Beispiel 1 erhält man die gereinigte Titelverbindung als zähes Öl; MS (+FAB-Methode): m/z (rel. Int. [%]) = 643 ($[M+H]^+$, 7), 229 (54), 154 ([m-$NO_2$-BenzylOH] 100); IR (KBr): 2165 $cm^{-1}$ (C≡N). Zur weiteren Analytik wird ein Teil der Substanz in das Salz der Weinsäure überführt, das unter reduziertem Druck einen Trockenschaum bildet.

Beispiel 60: N-Cyano-N′-[6-[N-[2-[N-(4-fluorbenzyl)-N-(2-pyridyl)amino]ethyl]-N-methylamino]hexyl]-N″-[3-[3-(piperidinomethyl)phenoxy]propyl]guanidin

Die Herstellung erfolgt analog Beispiel 1 ausgehend von 0,33 g (0,9 mmol) N-[2-[N-(4-Fluorbenzyl)-N-(2-pyridyl)amino]ethyl]-N-methyl-1,6-hexandiamin und der äquimolaren Menge N-Cyano-O-phenyl-N′-[3-(3-(piperidinomethyl)phenoxy]propyl]isoharnstoff. Nach chromatographischer Aufarbeitung analog Beispiel 1 erhält man die gereinigte Titelverbindung als zähes Öl; MS (+FAB-Methode): m/z (rel. Int. [%]) = 657 ($[M+H]^+$, 11), 229 (100); IR (KBr): 2164 $cm^{-1}$ (C≡N). Zur weiteren Analytik wird ein Teil der Substanz in das Salz der Weinsäure in einem Lösungsmittelgemisch Ethanol/Ether überführt; Smp.: 58°C (Ethanol/Ether).

Beispiel 61: N-Cyano-N′-[7-[N-[2-[N-(4-fluorbenzyl)-N-(2-pyridyl)amino]ethyl]-N-methylamino]heptyl]-N″-[3-[3-(piperidinomethyl)phenoxy]propyl]guanidin

Die Herstellung erfolgt analog Beispiel 1 ausgehend von 0,48 g (1,3 mmol) N-[2-[N-(4-Fluorbenzyl)-N-(2-pyridyl)amino]ethyl]-N-methyl-1,7-heptandiamin und der äquimolaren Menge N-Cyano-O-phenyl-N′-[3-[3-(piperidinomethyl)phenoxy]propyl]isoharnstoff. Nach chromatographischer Aufarbeitung analog Beispiel 1 erhält man die gereinigte Titelverbindung als zähes Öl; MS (+FAB-Methode): m/z (rel. Int. [%]) = 671 ($[M+H]^+$, 3), 229 (100); IR (KBr): 2164 $cm^{-1}$ (C≡N). Zur weiteren Analytik wird ein Teil der Substanz in das Salz der O,O′-Ditoluoylweinsäure überführt und aus Isopropanol/Ether umkristallisiert; Smp.: 96-98°C (Isopropanol/Ether).

Beispiel 62: N-Cyano-N′-[3-[N-[2-[N-(4-fluorbenzyl)-N-(2-pyridyl)amino]ethyl]-N-methylamino]propyl]-N″-[4-[3-(piperidinomethyl)phenoxy]butyl]guanidin

Die Herstellung erfolgt analog Beispiel 1 ausgehend von 0,50 g (1,6 mmol) N-[2-[N-(4-Fluorbenzyl)-N-(2-pyridyl)amino]ethyl]-N-methyl-1,3-propandiamin und der äquimolaren Menge N-Cyano-O-phenyl-N′-[4-[3-(piperidinomethyl)phenoxy]butyl]isoharnstoff. Nach chromatographischer Aufarbeitung analog Beispiel 1 erhält man die gereinigte Titelverbindung als zähes Öl; MS (+FAB-Methode): m/z (rel. Int. [%]) = 629 ($[M+H]^+$, 18), 229 (98), 109 (100); IR (KBr): 2164 $cm^{-1}$ (C≡N). Zur weiteren Analytik wird ein Teil der Substanz in das Salz der O,O′-Ditoluoylweinsäure überführt und aus Isopropanol/Ether umkristallisiert; Smp.: 102-105°C (Isopropanol/Ether).

Beispiel 63: N-[2-[N-[2-[N-(4-Methoxybenzyl)-N-(2-pyridyl)amino]ethyl]-N-methylamino]ethyl]-N′-[3-[3-(piperidinomethyl)phenoxyl]propyl]harnstoff

0,53 g (1,7 mmol) N-[2-[N-(4-Methoxybenzyl)-N-(2-pyridyl)amino]-ethyl]-N-methyl-1,2-ethandiamin werden in 2,5ml absolutem THF gelöst und zu einer auf 0°C gekühlten Lösung von 0,27 g (1,7 mmol) 1,1′-Carbonyldiimidazol in 9 ml absolutem THF unter Rühren innerhalb 30 Minuten so zugetropft, dass die Temperatur des Reaktionsansatzes bei 0°C bleibt. Die Lösung wird solange bei 0°C weitergerührt bis sich das Amin vollständig zum Isocyanat umgesetzt hat (DC-Kontrolle). Zu der Lösung des Isocyanats wird eine äquimolare Lösung von 3-[3-(Piperidinomethyl)phenoxy]propylamin in 5ml absolutem THF zugetropft. Der Reaktionsansatz wird 24 Stunden bei Raumtemperatur gerührt. Auf den Ansatz wird anschliessend Wasser gegeben, 30 Minuten gerührt und mit Methylenchlorid extrahiert. Die organischen Phasen werden vereinigt, mit $NA_2SO_4$ getrocknet und das Lösungsmittel unter verminderten Druck abdestilliert. Der Rückstand wird rotationschromatograpisch gereinigt und man erhält die gereinigte Titelverbindung als zähes Öl.
MS (+FAB-Methode): m/z (rel. Int. [%]) = 589 ($[M+H]^+$, 1), 241 (10), 121 (100)
IR (KBr): 1639 $cm^{-1}$ (C=O)

Beispiel 64: N-[3-[N-[2-[N-[4-Methoxybenzyl)-N-(2-pyridyl)amino]ethyl]-N-methylamino]propyl]-N′-[3-[3-(piperidinomethyl)phenoxy]propyl]harnstoff

Die Herstellung erfolgt analog Beispiel 63 ausgehend von 0,54 g (1,65 mmol) N-[2-[N-(4-Methoxybenzyl)-N-(2-pyridyl)amino]ethyl]-N-methyl-1,3-propandiamin und der äquimolaren Mengen 1,1′-Carbonyldiimidazol und 3-[3-(Piperidinomethyl)phenoxy]propylamin. Nach chromatographischer Aufarbeitung analog Beispiel 63 erhält man die gereinigte Titelverbindung als zähes Öl; MS (+FAB-Methode): m/z (rel. Int. [%]) = 603 ($[M+H]^+$,

10), 121 (100); IR (KBr): 1639 $cm^{-1}$ (C=O). Zur weiteren Analytik wird ein Teil der Substanz in das Salz der O,O'-Ditoluoylweinsäure überführt und aus Isopropanol/Ether umkristallisiert; Smp.: 120°C (Isopropanol/Ether).

Beispiel 65: N-[4-[N-[2-[N-(4-Methoxybenzyl)-N-(2-pyridyl)amino]ethyl]-N-methylamino]butyl]-N'-[3-[3-(piperidinomethyl)phenoxy]propyl]harnstoff

Die Herstellung erfolgt analog Beispiel 63 ausgehend von 0,55 g (1,6 mmol) N-[2-[N-(4-Methoxybenzyl)-N-(2-pyridyl)amino]ethyl]-N-methyl-1,4-butandiamin und der äquimolaren Mengen 1,1'-Carbonyldiimidazol und 3-[3-(Piperidinomethyl)phenoxy]propylamin. Nach chromatographischer Aufarbeitung analog Beispiel 63 erhält man die gereinigte Titelverbindung als zähes Öl; MS (+FAB-Methode): m/z (rel. Int. [%]) = 617 ($[M+H]^+$, 1), 121 (100); IR (KBr): 1603 $cm^{-1}$ (C=O). Zur weiteren Analytik wird ein Teil der Substanz in das Salz der Pikrinsäure überführt und aus Ethanol umkristallisiert; Smp.: 68-72°C (Ethanol).

Beispiel 66: N-[6-[N-[2-[N-(4-Methoxybenzyl)-N-(2-pyridyl)amino]ethyl]-N-methylamino]hexyl]-N'-[3-[3-(piperidinomethyl)phenoxy]propyl]harnstoff

Die Herstellung erfolgt analog Beispiel 63 ausgehend von 0,58 g (1,5 mmol) N-[2-[N-(4-Methoxybenzyl)-N-(2-pyridyl)amino]ethyl]-N-methyl-1,6-hexandiamin und der äquimolaren Mengen 1,1'-Carbonyldiimidazol und 3-[3-(Piperidinomethyl)phenoxy]propylamin. Nach chromatographischer Aufarbeitung analog Beispiel 63 erhält man die gereinigte Titelverbindung als zähes Öl; MS (+FAB-Methode): m/z (rel. Int. [%]) = 645 ($[M+H]^+$, 8), 121 (100); IR (KBr): 1635 $cm^{-1}$(C=O). Zur weiteren Analytik wird ein Teil der Substanz in das Salz der Weinsäure in einem Lösungsmittelgemisch Ethanol/Ether überführt; Smp.: 75°C (Ethanol/Ether).

Beispiel 67: N-[2-[N-[2-[N-(4-Fluorbenzyl)-N-(2-pyridyl)amino]ethyl]-N-methylamino]ethyl-N'-[3-[3-(piperidinomethyl)phenoxy]propyl]harnstoff

Die Herstellung erfolgt analog Beispiel 63 ausgehend von 0,42 g (1,0 mmol) N-[2-[N-(4-Fluorbenzyl)-N-(2-pyridyl)amino]ethyl]-N-methyl-1,2-ethandiamin und der äquimolaren Mengen 1,1'-Carbonyldiimidazol und 3-[3-(Piperidinomethyl)phenoxy]propylamin. Nach chromatographischer Aufarbeitung analog Beispiel 63 erhält man die gereinigte Titelverbindung als zähes Öl; MS (+FAB-Methode): m/z (rel. Int. [%]) = 577 ($[M+H]^+$, 12), 229 (99), 109 (100); IR (KBr): 1640 $cm^{-1}$ (C=O). Zur weiteren Analytik wird ein Teil der Substanz in das Salz der Weinsäure überführt und aus Isopropanol/Ether/Petrolether umkristallisiert; Smp.: 68°C (Isopropanol/Ether/Petrolether).

Beispiel 68: N-[3-[N-[2-[N-(4-Fluorbenzyl)-N-(2-pyridyl)amino]ethyl]-N-methylamino]propyl]-N'-[3-[3-(piperidinomethyl)phenoxy]propyl]harnstoff

Die Herstellung erfolgt analog Beispiel 63 ausgehend von 0,63 g (2,0 mmol) N-[2-[N-(4-Fluorbenzyl)-N-(2-pyridyl)amino]ethyl]-N-methyl-1,3-propandiamin und der äquimolaren Mengen 1,1'-Carbonyldiimidazol und 3-[3-(Piperidinomethyl)phenoxy]propylamin. Nach chromatographischer Aufarbeitung analog Beispiel 63 erhält man die gereinigte Titelverbindung als zähes Öl; MS (+FAB-Methode): m/z (rel. Int. [%]) = 591 ($[M+H]^+$, 7), 229 (86), 109 (100). Zur weiteren Analytik wird ein Teil der Substanz in das Salz der Salzsäure überführt und als Trockenschaum aus Ether ausgerührt; Smp.: 123-125°C (Ether).

Beispiel 69: N-[4-[N-[2-[N-(4-Fluorbenzyl)-N-(2-pyridyl)amino]ethyl]-N-methylamino]butyl]-N'-[3-[3-(piperidinomethyl)phenoxy]propyl]harnstoff

Die Herstellung erfolgt analog Beispiel 63 ausgehend von 0,45 g (1,0 mmol) N-[2-[N-(4-Fluorbenzyl)-N-(2-pyridyl)amino]ethyl]-N-methyl-1,4-butandiamin und der äquimolaren Mengen 1,1'-Carbonyldiimidazol und 3-[3-(Piperidinomethyl)phenoxy]propylamin. Nach chromatographischer Aufarbeitung analog Beispiel 63 erhält man die gereinigte Titelverbindung als zähes Öl; MS (+FAB-Methode): m/z (rel. Int. [%]) = 605 ($[M+H]^+$, 8), 109 (100); IR (KBr): 1641 $cm^{-1}$ (C=O). Zur weiteren Analytik wird ein Teil der Substanz in das Salz der Weinsäure überführt und aus Isopropanol/Ether/Petrolether umkristallisiert; Smp.: 69°C (Isopropanol/Ether/Petrolether).

Beispiel 70: N-[5-[N-[2-[N-(4-Fluorbenzyl)-N-(2-pyridyl)amino]ethyl]-N-methylamino]pentyl]-N′-[3-[3-(piperidinomethyl)phenoxy]propyl]harnstoff

Die Herstellung erfolgt analog Beispiel 63 ausgehend von 0,51 g (1,5 mmol) N-[2-[N-(4-Fluorbenzyl)-N-(2-pyridyl)amino]ethyl]-N-methyl-1,5-pentandiamin und der äquimolaren Mengen 1,1′-Carbonyldiimidazol und 3-[3-(Piperidinomethyl)phenoxy]propylamin. Nach chromatographischer Aufarbeitung analog Beispiel 63 erhält man die gereinigte Titelverbindung als zähes Öl; MS (+FAB-Methode): m/z (rel. Int. [%]) = 619 ($[M+H]^+$, 3), 77 (100); IR (KBr): 1634 $cm^{-1}$ (C=O). Zur weiteren Analytik wird ein Teil der Substanz in das Salz der Weinsäure überführt und aus Isopropanol/Ether/Petrolether umkristallisiert; Smp.: 63°C (Isopropanol/Ether/Petrolether).

Beispiel 71: N-[6-[N-[2-[N-(4-Fluorbenzyl)-N-(2-pyridyl)amino]ethyl]-N-methylamino]hexyl]-N′-[3-[3-(piperidinomethyl)phenoxy]propyl]harnstoff

Die Herstellung erfolgt analog Beispiel 63 ausgehend von 0,50 g (1,4 mmol) N-[2-[N-(4-Fluorbenzyl)-N-(2-pyridyl)amino]ethyl]-N-methyl-1,6-hexandiamin und der äquimolaren Mengen 1,1′-Carbonyldiimidazol und 3-[3-(Piperidinomethyl)phenoxy]propylamin. Nach chromatographischer Aufarbeitung analog Beispiel 63 erhält man die gereinigte Titelverbindung als zähes Öl; MS (+FAB-Methode): m/z (rel. Int. [%]) = 633 ($[M+H]^+$, 1), 154 ([m-$NO_2$-BenzylOH] 100); IR (KBr): 1640 $cm^{-1}$ (C=O). Zur weiteren Analytik wird ein Teil der Substanz in das Salz der Weinsäure in einem Lösungsmittelgemisch Ethanol/Ether überführt; Smp.: 65-69°C (Ethanol/Ether).

Beispiel 72: N-[7-[N-[2-[N-[4-Fluorbenzyl)-N-(2-pyridyl)amino]ethyl]-N-methylamino]heptyl]-N′-[3-[3-(piperidinomethyl)phenoxy]propyl]harnstoff

Die Herstellung erfolgt analog Beispiel 63 ausgehend von 0,59 g (1,6 mmol) N-[2-[N-(4-Fluorbenzyl)-N-(2-pyridyl)amino]ethyl]-N-methyl-1,7-heptandiamin und der äquimolaren Mengen 1,1′-Carbonyldiimidazol und 3-[3-(Piperidinomethyl)phenoxy]propylamin. Nach chromatographischer Aufarbeitung analog Beispiel 63 erhält man die gereinigte Titelverbindung als zähes Öl; MS (+FAB-Methode): m/z (rel. Int. [%]) = 647 ($[M+H]^+$, 7), 229 (100); IR (KBr): 1634 $cm^{-1}$ (C=O). Zur weiteren Analytik wird ein Teil der Substanz in das Salz der O,O′-Ditoluoylweinsäure überführt und aus Isopropanol/Ether umkristallisiert; Smp.: 112°C (Isopropanol/Ether).

Beispiel 73: N-[3-[N-[2-[N-(4-Fluorbenzyl)-N-(2-pyridyl)amino]ethyl]-N-methylamino]propyl]-N′-[4-[3-(piperidinomethyl)phenoxy]butyl]harnstoff

Die Herstellung erfolgt analog Beispiel 63 ausgehend von 0,42 g (1,0 mmol) N-[2-[N-(4-Fluorbenzyl)-N-(2-pyridyl)amino]ethyl]-N-methyl-1,3-propandiamin und der äquimolaren Mengen 1,1′-Carbonyldiimidazol und 4-[3-(Piperidinomethyl)phenoxy]butylamin. Nach chromatographischer Aufarbeitung analog Beispiel 63 erhält man die gereinigte Titelverbindung als zähes Öl; MS (+FAB-Methode): m/z (rel. Int. [%]) = 605 ($[M+H]^+$, 14), 229 (100); IR (KBr): 1631 $cm^{-1}$ (C=O). Zur weiteren Analytik wird ein Teil der Substanz in das Salz der O,O′-Ditoluoylweinsäure überführt und aus Isopropanol/Ether umkristallisiert; Smp.: 115°C (Isopropanol/Ether).

Beispiel 74: N-[2-[N-[2-[N-(4-Methoxybenzyl)-N-(2-pyridyl)amino]ethyl]-N-methylamino]ethyl]-N′-[3-[3-(piperidinomethyl)phenoxy]propyl]-2-nitro-1,1-ethendiamin

Die Herstellung erfolgt analog Beispiel 22 ausgehend von 0,85 g (2,7 mmol) N-[2-[N-(4-Methoxybenzyl)-N-(2-pyridyl)amino]ethyl]-N-methyl-1,2-ethandiamin und der äquimolaren Menge 1-Methylthio-1-[3-[3-(piperidinomethyl)phenoxy]propyl]amino-2-nitro-ethen. Nach chromatographischer Aufarbeitung analog Beispiel 22 erhält man die gereinigte Titelverbindung als zähes Öl; MS (+FAB-Methode): m/z (rel. Int. [%]) = 632 ($[M+H]^+$, 8), 121 (100). Zur weiteren Analytik wird ein Teil der Substanz in das Salz der Weinsäure überführt und aus Isopropanol/Ether umkristallisiert; Smp.: 102-104°C (Isopropanol/Ether).

Beispiel 75: N-[6-[N-[2-[N-(4-Methoxybenzyl)-N-(2-pyridyl)amino]ethyl]-N-methylamino]hexyl]-N′-[3-[3-(piperidinomethyl)phenoxy]propyl]-2-nitro-1,1-ethendiamin

Die Herstellung erfolgt analog Beispiel 22 ausgehend von 0,54 g (1,45 mmol) N-[2-[N-(4-Methoxybenzyl)-N-(2-pyridyl)amino]ethyl]-N-methyl-1,6-hexa ndiamin und der äquimolaren Menge 1-Methylthio-1-[3-[3-(piperidinomethyl)phenoxy]propyl]amino-2-nitro-ethen. Nach chromatographischer Aufarbeitung analog Beispiel 22 erhält man die gereinigte Titelverbindung als zähes Öl; MS (+FAB-Methode): m/z (rel. Int. [%]) = 688

([M+H]$^+$, 5), 121 (100). Zur weiteren Analytik wird ein Teil der Substanz in das Salz der Weinsäure in einem Lösungsmittelgemisch Ethanol/Ether überführt; Smp.: 73°C (Ethanol/Ether).

Beispiel 76: N-Cyano-N′-[2-[[(2-guanidino-4-thiazolyl)methyl]thio]ethyl]-N″-[2-[N-[2-[N-(4-methoxybenzyl)-N-(2-pyridyl)amino]ethyl]-N-methylamino]ethyl]guanidin

Die Herstellung erfolgt analog Beispiel 1 ausgehend von 0,53 g (1,7 mmol) N-[2-[N-[4-Methoxybenzyl)-N-(2-pyridyl)amino]ethyl]-N-methyl-1,2-ethandiamin und der äquimolaren Menge N-Cyano-N′-[2-[[(2-guanidino-4-thiazolyl)methyl]thio]ethyl]-O-phenylisoharnstoff. Nach chromatographischer Aufarbeitung analog Beispiel 1 erhält man die gereinigte Titelverbindung als zähes Öl, das aus absolutem Ether bei -20°C auskristallisiert und aus Ethanol/Ether umkristallisiert wird; MS (+FAB-Methode):m/z (rel. Int. [%]) = 596 ([M+H]$^+$, 1), 121 (100); IR (KBr): 2163 $cm^{-1}$ (C≡N); Smp.: 56-58°C (Ethanol/Ether).

Beispiel 77: N-Cyano-N′-[2-[[(2-guanidino-4-thiazolyl)methyl]thio]ethyl]-N″-[3-[N-[2-[N-(4-methoxybenzyl)-N-(2-pyridyl)amino]ethyl]-N-methylamino]propyl]guanidin

Die Herstellung erfolgt analog Beispiel 1 ausgehend von 0,52 g (1,6 mmol) N-[2-[N-(4-Methoxybenzyl)-N-(2-pyridyl)amino]ethyl]N-methyl-1,3-propandiamin und der äquimolaren Menge N-Cyano-N′-[2-[[(2-guanidino-4-thiazolyl)methyl]thio]ethyl]-O-phenylisoharnstoff. Nach chromatographischer Aufarbeitung analog Beispiel 1 erhält man die gereinigte Titelverbindung als zähes Öl, das aus Ether bei -20°C auskristallisiert; MS (+FAB-Methode): m/z (rel. Int. [%]) = 610 ([M+H]$^+$, 1), 121 (100); IR (KBr): 2161 $cm^{-1}$ (C≡N); Smp.: 54°C (Ether).

Beispiel 78: N-Cyano-N′-[2-[[(2-guanidino-4-thiazolyl)methyl]thio]ethyl]-N″-[4-[N-[2-[N-(4-methoxybenzyl)-N-(2-pyridyl)amino]ethyl]-N-methylamino]butyl]guanidin

Die Herstellung erfolgt analog Beispiel 1 ausgehend von 0,54 g (1,6 mol) N-[2-[N-(4-Methoxybenzyl)-N-(2-pyridyl)amino]ethyl]-N-methyl-1,4-butandiamin und der äquimolaren Menge N-Cyano-N′-[2-[[(2-guanidino-4-thiazolyl)methyl]thio]ethyl]-O-phenyl-isoharnstoff. Nach chromatographischer Aufarbeitung analog Beispiel 1 erhält man die gereinigte Titelverbindung als Trockenschaum.
MS (+FAB-Methode): m/z (rel. Int. [%]) = 624 ([M+H]$^+$, 1), 121 (100)
IR (KBr): 2163 $cm^{-1}$ (C≡N)

Beispiel 79: N-Cyano-N′-[2-[[(2-guanidino-4-thiazolyl)methyl]thio]ethyl]-N″-[6-[N-[2-[N-(4-methoxybenzyl)-N-(2-pyridyl)amino]ethyl]-N-methylamino]hexyl]guanidin

Die Herstellung erfolgt analog Beispiel 1 ausgehend von 0,57 g (1,5 mmol) N-[2-[N-(4-Methoxybenzyl)-N-(2-pyridyl)amino]ethyl]-N-methyl-1,6-hexandiamin und der äquimolaren Menge N-Cyano-N′-[2-[[(2-guanidino-4-thiazolyl)methyl]thio]ethyl]-O-phenylisoharnstoff. Nach chromatographischer Aufarbeitung analog Beispiel 1 erhält man die gereinigte Titelverbindung als zähes Öl, das aus Ether bei -20°C auskristallisiert; MS (+FAB-Methode): m/z (rel. Int. [%]) = 652 ([M+H]$^+$, 12), 121 (100); IR (KBr): 2162 $cm^{-1}$ (C≡N); Smp.: 52°C (Ether).

Beispiel 80: N-Cyano-N′-[2-[N-[2-[N-(4-fluorbenzyl)-N-(2-pyridyl)amino]ethyl]-N-methylamino]ethyl]-N″-[2-[[(2-guanidino-4-thiazolyl)methyl]thio]ethyl]guanidin

Die Herstellung erfolgt analog Beispiel 1 ausgehend von 0,30 g (1,0 mmol) N-[2-[N-(4-Fluorbenzyl)-N-(2-pyridyl)amino]ethyl]-N-methyl-1,2-ethandiamin und der äquimolaren Menge N-Cyano-N′-[2-[[(2-guanidino-4-thiazolyl)methyl]thio]ethyl]-O-phenyl-isoharnstoff. Nach chromatographischer Aufarbeitung analog Beispiel 1 erhält man die gereinigte Titelverbindung als zähes Öl; MS (+FAB-Methode): m/z (rel. Int. [%]) = 584 ([M+H]$^+$, 3), 109 (100); IR (KBr): 2165 $cm^{-1}$ (C≡N). Zur weiteren Analytik wird ein Teil der Substanz in das Salz der Weinsäure überführt und aus Isopropanol/Ether umkristallisiert; Smp.: 109°C (Isopropanol/Ether).

Beispiel 81: N-Cyano-N′-[3-[N-[2-[N-(4-fluorbenzyl)-N-(2-pyridyl)amino]ethyl]-N-methylamino]propyl]-N″-[2-[[(2-guanidino-4-thiazolyl)methyl]thio]ethyl]guanidin

Die Herstellung erfolgt analog Beispiel 1 ausgehend von 0,53 g (1,67 mmol) N-[2-[N-(4-Fluorbenzyl)-N-(2pyridyl)amino]ethyl]-N-methyl-1,3-propandiamin und der äquimolaren Menge N-Cyano-N′-[2-[[(2-guanidino-4-thiazolyl)methyl]thio]ethyl]-O-phenyl-isoharnstoff. Nach chromatographischer Aufarbeitung analog Beispiel 1 erhält man die gereinigte Titelverbindung als zähes Öl.

MS (+FAB-Methode): m/z (rel. Int. [%]) = 598 ([M+H]$^+$, 6), 109 (100)
IR (KBr): 2163 $cm^{-1}$ (C≡N) $C_{27}H_{36}FN_{11}S_2$ (597,80)

Beispiel 82: N-Cyano-N'-[4-[N-[2-[N-(4-fluorbenzyl)-N-(2-pyridyl)amino]ethyl]-N-methylamino]butyl]-N''-[2-[[(2-guanidino-4-thiazolyl)methyl]thio]ethyl]guanidin

Die Herstellung erfolgt analog Beispiel 1 ausgehend von 0,33 g (1,0 mmol) N-[2-[N-(4-Fluorbenzyl)-N-(2-pyridyl)amino]ethyl]-N-methyl-1,4-butandiamin und der äquimolaren Menge N-Cyano-N'-[2-[[(2-guanidino-4-thiazolyl)methyl]thio]ethyl]-O-phenyl-isoharnstoff. Nach chromatographischer Aufarbeitung analog Beispiel 1 erhält man die gereinigte Titelverbindung als zähes Öl; MS (+FAB-Methode): m/z (rel. Int. [%]) = 612 ([M+H]$^+$, 49), 229 (100); IR (KBr): 2165 $cm^{-1}$ (C≡N). Zur weiteren Analytik wird ein Teil der Substanz in das Salz der O,O'-Ditoluoylweinsäure in einem Lösungsmittelgemisch Ethanol/Ether überführt; Smp.: 137°C (Ethanol/Ether).

Beispiel 83: N-Cyano-N'-[5-[N-[2-[N-(4-fluorbenzyl)-N-(2-pyridyl)amino]ethyl]-N-methylamino]pentyl]-N''-[2-[[(2-guanidino-4-thiazolyl)methyl]thio]ethyl]guanidin

Die Herstellung erfolgt analog Beispiel 1 ausgehend von 0,51 g (1,5 mmol) N-[2-[N-(4-Fluorbenzyl)-N-(2-pyridyl)amino]ethyl]-N-methyl-1,5-pentandiamin und der äquimolaren Menge N-Cyano-N'-[2-[[(2-guanidino-4-thiazolyl)methyl]thio]ethyl]-O-phenyl-isoharnstoff. Nach chromatographischer Aufarbeitung analog Beispiel 1 erhält man die gereinigte Titelverbindung als zähes Öl; MS (+FAB-Methode): m/z (rel. Int. [%]) = 626 ([M+H]$^+$, 3), 154 ([m-$NO_2$BenzylOH] 100); IR (KBr): 2162 $cm^{-1}$ (C≡N). Zur weiteren Analytik wird ein Teil der Substanz in das Salz der Weinsäure überführt und aus Isopropanol/Petrolether umkristallisiert; Smp.: 103°C (Isopropanol/Petrolether).

Beispiel 84: N-Cyano-N'-[6-[N-[2-[N-(4-fluorbenzyl)-N-(2-pyridyl)amino]ethyl]-N-methylamino]hexyl]-N''-[2-[[(2-guanidino-4-thiazolyl)methyl]thio]ethyl]guanidin

Die Herstellung erfolgt analog Beispiel 1 ausgehend von 0,50 g (1,4 mmol) N-[2-[N-(4-Fluorbenzyl)-N-(2-pyridyl)amino]ethyl]-N-methyl-1,6-hexandiamin und der äquimolaren Menge N-Cyano-N'-[2-[[(2-guanidino-4-thiazolyl)methyl]thio]ethyl]-O-phenyl-isoharnstoff. Nach chromatographischer Aufarbeitung analog Beispiel 1 erhält man die gereinigte Titelverbindung als zähes Öl; MS (+FAB-Methode): m/z (rel. Int. [%]) = 639 ([M+H]$^+$, 9), 229 (100); IR (KBr): 2162 $cm^{-1}$ (C≡N). Zur weiteren Analytik wird ein Teil der Substanz in das Salz der Weinsäure in einem Lösungsmittelgemisch Ethanol/Ether überführt; Smp.: 75°C (Ethanol/Ether).

Beispiel 85: N-Cyano-N'-[7-[N-[2-[N-(4-fluorbenzyl)-N-(2-pyridyl)amino]ethyl]-N-methylamino]heptyl]-N''-[2-[[(2-guanidino-4-thiazolyl)methyl]thio]ethyl]guanidin

Die Herstellung erfolgt analog Beispiel 1 ausgehend von 0,57 g (1,53 mmol) N-[2-[N-(4-Fluorbenzyl)-N-(2-pyridyl)amino]ethyl]-N-methyl-1,7-heptandiamin und der äquimolaren Menge N-Cyano-N'-[2-[[(2-guanidino-4-thiazolyl)methyl]thio]ethyl]-O-phenyl-isoharnstoff. Nach chromatographischer Aufarbeitung analog Beispiel 1 erhält man die gereinigte Titelverbindung als zähes Öl; MS (+FAB-Methode): m/z (rel. Int. [%]) = 654 ([M+H]$^+$, 2), 91 (100); IR (KBr): 2163 $cm^{-1}$ (C≡N). Zur weiteren Analytik wird ein Teil der Substanz in das Salz der O,O'-Ditoluoylweinsäure in einem Lösungsmittelgemisch Ethanol/Ether überführt; Smp.: 118°C (Ethanol/Ether).

Beispiel 86: N-[2-[[(2-Guanidino-4-thiazolyl)methyl]thio]ethyl]-N'-[2-[N-[2-[N-(4-methoxybenzyl)-N-(2-pyridyl)amino]ethyl]-N-methylamino]ethyl]harnstoff

Die Herstellung erfolgt analog Beispiel 63 ausgehend von 0,53 g (1,7 mmol) N-[2-[N-(4-Methoxybenzyl)-N-(2-pyridyl)amino]ethyl]-N-methyl-1,2-ethandiamin und der äquimolaren Mengen von 1,1'-Carbonyldiimidazol und 2-[[(2-Guanidino-4-thiazolyl)methyl]thio]ethanamin. Nach chromatographischer Aufarbeitung analog Beispiel 63 erhält man die gereinigte Titelverbindung als zähes Öl; MS (+FAB-Methode): m/z (rel. Int. [%]) = 572 ([M+H]$^+$, 1), 121 (100); IR (KBr): 1644 $cm^{-1}$ (C=O). Zur weiteren Analytik wird ein Teil der Substanz in das Salz der Pikrinsäure überführt und aus Ethanol umkristallisiert; Smp.: 84°C (Ethanol).

Beispiel 87: N-[2-[[(2-Guanidino-4-thiazolyl)methyl]thio]ethyl]-N′-[3-[N-[2-[N-(4-methoxybenzyl)-N-(2-pyridyl)amino]ethyl]-N-methylamino]propyl]harnstoff

Die Herstellung erfolgt analog Beispiel 63 ausgehend von 1,00 g (3,0 mmol) N-[2-[N-(4-Methoxybenzyl)-N-(2-pyridyl)amino]ethyl]-N-methyl-1,3-propandiamin und der äquimolaren Mengen von 1,1′-Carbonyldiimidazol und 2-[[(2-Guanidino-4-thiazolyl)methyl]thio]ethanamin. Nach chromatographischer Aufarbeitung analog Beispiel 63 erhält man die gereinigte Titelverbindung als zähes Öl; MS (+FAB-Methode): m/z (rel. Int. [%]) = 586 ($[M+H]^+$, 4), 121 (100); IR (KBr): 1717 $cm^{-1}$ (C=O). Zur weiteren Analytik wird ein Teil der Substanz in das Salz der Pikrinsäure überführt und aus Ethanol umkristallisiert; Smp.: 106-109°C (Ethanol).

Beispiel 88: N-[2-[[(2-Guanidino-4-thiazolyl)methyl]thio]ethyl]-N′-[4-[N-[2-[N-(4-methoxybenzyl)-N-(2-pyridyl)amino]ethyl]-N-methylamino]butyl]harnstoff

Die Herstellung erfolgt analog Beispiel 63 ausgehend von 0,54 g (1,58 mmol) N-[2-[N-(4-Methoxybenzyl)-N-(2-pyridyl)amino]ethyl]-N-methyl-1,4-butandiamin und der äquimolaren Mengen von 1,1′-Carbonyldiimidazol und 2-[[(2-Guanidino-4-thiazolyl)methyl]thio]ethanamin. Nach chromatographischer Aufarbeitung analog Beispiel 63 erhält man die gereinigte Titelverbindung als zähes Öl; MS (+FAB-Methode): m/z (rel. Int. [%]) = 600 ($[M+H]^+$, 1), 121 (100); IR (KBr): 1599 $cm^{-1}$ (C=O). Zur weiteren Analytik wird ein Teil der Substanz in das Salz der Pikrinsäure überführt und aus Ethanol umkristallisiert; Smp.: 104-106°C (Ethanol).

Beispiel 89: N-[2-[[(2-Guanidino-4-thiazolyl)methyl]thio]ethyl]-N′-[6-[N-[2-[N-(4-methoxybenzyl)-N-(2-pyridyl)amino]ethyl]-N-methylamino]hexyl]harnstoff

Die Herstellung erfolgt analog Beispiel 63 ausgehend von 0,74 g (2,0 mmol) N-[2-[N-(4-Methoxybenzyl)-N-(2-pyridyl)amino]ethyl]-N-methyl-1,6-hexandiamin und der äquimolaren Mengen von 1,1′-Carbonyldiimidazol und 2-[[(2-Guanidino-4-thiazolyl)methyl]thio]ethanamin. Nach chromatographischer Aufarbeitung analog Beispiel 63 erhält man die gereinigte Titelverbindung als zähes Öl; MS (+FAB-Methode): m/z (rel. Int. [%]) = 628 ($[M+H]^+$, 4), 121 (100); IR (KBr): 1597 $cm^{-1}$ (C=O). Zur weiteren Analytik wird ein Teil der Substanz in das Salz der Weinsäure in einem Lösungsmittelgemisch Ethanol/Ether überführt; Smp.: 60°C (Ethanol/Ether).

Beispiel 90: N-[2-[N-[2-[N-(4-Fluorbenzyl)-N-(2-pyridyl)amino]ethyl]-N-methylamino]ethyl]-N′-[2-[[(2-guanidino-4-thiazolyl)methyl]thio]ethyl]harnstoff

Die Herstellung erfolgt analog Beispiel 63 ausgehend von 0,29 g (0,97 mmol) N-[2-[N-(4-Fluorbenzyl)-N-(2-pyridyl)amino]ethyl]-N-methyl-1,2-ethandiam in und der äquimolaren Mengen von 1,1′-Carbonyldiimidazol und 2-[[(2-Guanidino-4-thiazolyl)methyl]thio]ethanamin. Nach chromatographischer Aufarbeitung analog Beispiel 63 erhält man die gereinigte Titelverbindung als zähes Öl; MS (+FAB-Methode): m/z (rel. Int. [%]) = 560 ($[M+H]^+$, 8), 78 (100); IR (KBr): 1659 $cm^{-1}$ (C=O). Zur weiteren Analytik wird ein Teil der Substanz in das Salz der O,O′-Ditoluoylweinsäure in einem Lösungsmittelgemisch Ethanol/Ether überführt; Smp.: 132°C (Ethanol/Ether).

Beispiel 91: N-[3-[N-[2-[N-(4-Fluorbenzyl)-N-(2-pyridyl)amino]ethyl]-N-methylamino]propyl]-N′-[2-[[(2-guanidino-4-thiazolyl)methyl]thio]ethyl]harnstoff

Die Herstellung erfolgt analog Beispiel 63 ausgehend von 0,33 g (1,05 mmol) N-[2-[N-(4-Fluorbenzyl)-N-(2-pyridyl)amino]ethyl]-N-methyl-1,3-propandiamin und der äquimolaren Mengen von 1,1′-Carbonyldiimidazol und 2-[[(2-Guanidino-4-thiazolyl)methyl]thio]ethanamin. Nach chromatographischer Aufarbeitung analog Beispiel 63 erhält man die gereinigte Titelverbindung als Trockenschaum.
MS (+FAB-Methode): m/z (rel. Int. [%]) = 574 ($[M+H]^+$, 9), 109 (100)
IR (KBr): 1682 $cm^{-1}$ (C=O)

Beispiel 92: N-[4-[N-[2-[N-(4-Fluorbenzyl)-N-(2-pyridyl)amino]ethyl]-N-methylamino]butyl]-N′-[2-[[(2-guanidino-4-thiazolyl)methyl]thio]ethyl]harnstoff

Die Herstellung erfolgt analog Beispiel 63 ausgehend von 0,20 g (0,6 mmol) N-[2-[N-(4-Fluorbenzyl)-N-(2-pyridyl)amino]ethyl]-N-methyl-1,4-butandiamin und der äquimolaren Mengen von 1,1′-Carbonyldiimidazol und 2-[[(2-Guanidino-4-thiazolyl)methyl]thio]ethanamin. Nach chromatographischer Aufarbeitung analog Beispiel 63 erhält man die gereinigte Titelverbindung als zähes Öl.

MS (+FAB-Methode): m/z (rel. Int. [%]) = 588 ([M+H]$^+$, 2), 109 (100)
IR (KBr): 1685 $cm^{-1}$ (C=O) $C_{27}H_{38}FN_9OS_2$ (587,90)

Beispiel 93: N-[5-[N-[2-[N-(4-Fluorbenzyl)-N-(2-pyridyl)amino]ethyl]-N-methylamino]pentyl]-N'-[2-[[(2-guanidino-4-thiazolyl)methyl]thio]ethyl]harnstoff

Die Herstellung erfolgt analog Beispiel 63 ausgehend von 0,51 g (1,5 mmol) N-[2-[N-(4-Fluorbenzyl)-N-(2-pyridyl)amino]ethyl]-N-methyl-1,5-pentandiamin und der äquimolaren Mengen von 1,1'-Carbonyldiimidazol und 2-[[(2-Guanidino-4-thiazolyl)methyl]thio]ethanamin. Nach chromatographischer Aufarbeitung analog Beispiel 63 erhält man die gereinigte Titelverbindung als zähes Öl; MS (+FAB-Methode): m/z (rel. Int. [%]) = 602 ([M+H]$^+$, 3), 77 (100); IR (KBr): 1687 $cm^{-1}$ (C=O). Zur weiteren Analytik wird ein Teil der Substanz in das Salz der Weinsäure überführt und aus Isopropanol/Ether umkristallisiert; Smp.: 91-93°C (Isopropanol/Ether).

Beispiel 94: N-[6-[N-[2-[N-(4-Fluorbenzyl)-N-(2-pyridyl)amino]ethyl]-N-methylamino]hexyl]-N'-[2-[[(2-guanidino-4-thiazolyl)methyl]thio]ethyl]harnstoff

Die Herstellung erfolgt analog Beispiel 63 ausgehend von 0,50 g (1,4 mmol) N-[2-[N-(4-Fluorbenzyl)-N-(2-pyridyl)amino]ethyl]-N-methyl-1,6-hexandiamin und der äquimolaren Mengen von 1,1'-Carbonyldiimidazol und 2-[[(2-Guanidino-4-thiazolyl)methyl]thio]ethanamin. Nach chromatographischer Aufarbeitung analog Beispiel 63 erhält man die gereinigte Titelverbindung als zähes Öl; MS (+FAB-Methode): m/z (rel. Int. [%]) = 616 ([M+H]$^+$, 12), 229 (100); IR (KBr): 1640 $cm^{-1}$ (C=O). Zur weiteren Analytik wird ein Teil der Substanz in das Salz der Weinsäure in einem Lösungsmittelgemisch Ethanol/Ether überführt; Smp.: 95°C (Ethanol/Ether).

Beispiel 95: N-[7-[N-[2-[N-(4-Fluorbenzyl)-N-(2-pyridyl)amino]ethyl]-N-methylamino]heptyl]-N'-[2-[[(2-guanidino-4-thiazolyl)methyl]thio]ethyl]harnstoff

Die Herstellung erfolgt analog Beispiel 63 ausgehend von 0,59 g (1,6 mmol) N-[2-[N-(4-Fluorbenzyl)-N-(2-pyridyl)amino]ethyl]-N-methyl-1,7-heptandiamin und der äquimolaren Mengen von 1,1'-Carbonyldiimidazol und 2-[[(2-Guanidino-4-thiazolyl)methyl]thio]ethanamin. Nach chromatographischer Aufarbeitung analog Beispiel 63 erhält man die gereinigte Titelverbindung als zähes Öl; MS (+FAB-Methode): m/z (rel. Int. [%]) = 630 ([M+H]$^+$, 3), 109 (100); IR (KBr): 1666 $cm^{-1}$ (C=O). Zur weiteren Analytik wird ein Teil der Substanz in das Salz der O,O'-Ditoluoylweinsäure überführt und aus Isopropanol/Ether umkristallisiert; Smp.: 125°C (Isopropanol/Ether).

Beispiel 96: N-[2-[[(2-Guanidino-4-thiazolyl)methyl]thio]ethyl]-N'-[6-[N-[2-[N-(4-methoxybenzyl)-N-(2-pyridyl)amino]ethyl]-N-methylamino]hexyl]-2-nitro-1,1-ethendiamin

Die Herstellung erfolgt analog Beispiel 22 ausgehend von 0,41 g (1,8 mmol) 2-[[(2-Guanidino-4-thiazolyl)methyl]thio]ethanamin und der äquimolaren Menge 1-[6-[N-[2-[N-(4-Methoxybenzyl)-N-(2-pyridyl)amino]ethyl]-N-methylamino]hexyl]amino-1-methylthio-2-nitro-ethen. Nach chromatographischer Aufarbeitung analog Beispiel 22 erhält man die gereinigte Titelverbindung als zähes Öl; MS (+FAB-Methode): m/z (rel. Int. [%]) = 671 ([M+H]$^+$, 1), 121 (100). Zur weiteren Analytik wird ein Teil der Substanz in das Salz der Weinsäure in einem Lösungsmittelgemisch Ethanol/Ether überführt; Smp.: 60°C (Ethanol/Ether).

Die Ausgangsverbindung wird wie folgt hergestellt:

N-(4-Methoxybenzyl)-N'-methyl-N-(2-pyridyl)-1,2-ethandiamin wird mit der äquimolaren Menge 6-Bromhexannitril und der dreifach molaren Menge $Na_2CO_3$ in 60 ml wasserfreiem Acetonitril unter 3 Stunden Erhitzen unter Rückfluss umgesetzt. Nach Abkühlung auf Raumtemperatur wird soviel Wasser zum Reaktionsansatz gegeben bis sich der Niederschlag gelöst hat. Der Ansatz wird mit Toluol extrahiert. Die vereinigtenorganischen Phasen werden mit $MgSO_4$ getrocknet und das Toluol im Vakuum abdestilliert. Es resultiert N-[2-[N-(4-Methoxybenzyl)-N-(2-pyridyl)amino]ethyl]-N-methyl-6-aminohexannitril als bräunliches Öl. Dieses wird in 20 ml absolutem THF gelöst und unter Eiskühlung und Rühren langsam in eine Suspension der doppeltmolaren Menge von $LiAlH_4$ in 50 ml absolutem Ether eingetragen. Nach 30 min Rühren wird der Ansatz unter Eiskühlung mit wassergesättigtem Ether und tropfenweiser Zugabe von wenig 10 %iger Natronlauge hydrolysiert, bis die exotherme Reaktion beendet ist und sich ein weisser Niederschlag bildet. Man lässt weitere 30 min Rühren, saugt dann ab und wäscht den Niederschlag mehrmals mit Ether nach. Das Filtrat wird mit Wasser gewaschen, über $Na_2SO_4$ getrocknet und im Vakuum bis zur Trockne eingeengt. Das resultierende rohe Produkt wird unter Verwendung von Methlenchlorid mit 1 % (V/V) Methanol als Eluens dickschichtchromatographisch gereinigt. Man erhält N-[2-[N-(4-Methoxybenzyl)-N-(2-pyridyl)amino]ethyl]-N-methyl-1,6-hexandiamin in genügender Reinheit für die nachfolgende Reaktion. 0,43 g (1,16 mmol) dieses Amins werden mit 0,19 g (1,16 mmol) 1,1-Dimethylthio-2-nitroethen [R. Gomper, H. Schäfer, Chem. Ber. 100, 599 (1967)] in 20 ml absolutem Acetonitril 4 Stunden unter Rückfluss erhitzt. Nach Abschluss der Reaktion (dc-Kontrolle: Chloroform/Methanol 95/5 V/V) wird das Lösungsmittel im Vakuum abdestilliert. Das resultierende Produkt 1-[6-[N-[2-[N-(4-Methoxybenzyl)-N-(2-pyridyl)amino]ethyl]-N-methylamino]hexyl]amino-1-methylthio-2-nitroethen ist für die nachfolgende Reaktion rein genug.

Beispiel 97: N-[4-[N-[2-(Diphenylmethoxy)ethyl]-N-methylamino]butyl]-N′-[2-[[(2-guanidino-4-thiazolyl)methyl]thio]ethyl]harnstoff

Die Herstellung erfolgt analog Beispiel 63 ausgehend von 0,37 g (1,2 mmol) N-[2-(Diphenylmethoxy)ethyl]-N-methyl-1,4-butandiamin und der äquimolaren Mengen von 1,1′-Carbonyldiimidazol und 2-[[(2-Guanidino-4-thiazolyl)methyl]thio]ethanamin. Nach chromatographischer Aufarbeitung analog Beispiel 63 erhält man die gereinigte Titelverbindung als zähes Öl; MS (+FAB-Methode): m/z (rel. Int. [%]) = 570 ($[M+H]^+$, 11), 167 (100); IR (KBr): 1644 $cm^{-1}$ (C=O). Zur weiteren Analytik wird ein Teil der Substanz in das Salz der Weinsäure überführt und aus Acetonitril/Isopropanol/Petrolether umkristallisiert; Smp.: 101 °C (Acetonitril/Isopropanol/Petrolether).

Beispiel 98: N-[4-[N-(3′,3′-Diphenylpropyl)-N-methylamino]butyl]-N′-[2-[[(2-guanidino-4-thiazolyl)methyl]thio]ethyl]harnstoff

Die Herstellung erfolgt analog Beispiel 63 ausgehend von 0,53 g (1,8 mmol) N-(3′,3′-Diphenylpropyl)-N-methyl-1,4-butandiamin und der äquimolaren Mengen von 1,1′-Carbonyldiimidazol und 2-[[(2-Guanidino-4-thiazolyl)methyl]thio]ethanamin. Nach chromatographischer Aufarbeitung analog Beispiel 63 erhält man die gereinigte Titelverbindung als zähes Öl; MS (+FAB-Methode): m/z (rel. Int. [%]) = 554 ($[M+H]^+$, 2), 91 (100); IR (KBr): 1643 $cm^{-1}$ (C=O). Zur weiteren Analytik wird ein Teil der Substanz in das Salz der O,O′-Ditoluoylweinsäure in einem Lösungsmittelgemisch Ethanol/Ether überführt; Smp.: 136°C (Ethanol/Ether).

Beispiel 99: N-[2-[[(2-Guanidino-4-thiazolyl)methyl]thio]ethyl]-N′-[6-[N-[2-[N-(4-methoxybenzyl)-N-(2-pyridyl)amino]ethyl]-N-methylamino]hexyl]thioharnstoff

0,48 g (1,3 mmol) N-[2-[N-(4-Methoxybenzyl)-N-(2-pyridyl)amino]ethyl]-N-methyl-1,6-hexandiamin und 0,27 g (1,3 mmol) Dicyclohexylcarbodiimid werden bei -10°C in absolutem Ether mit 0,5ml Schwefelkohlenstoff gelöst, die Temperatur wird während 3 Stunden auf 20°C erhöht und weitere 12 Stunden gerührt. Der ausgefallene Feststoff wird abfiltriert und das Filtrat im Vakuum eingeengt. Der eingeengte Rückstand wird mit 0,30 g (1,3 mmol) 2-[[(2-Guanidino-4-thiazolyl)methyl]thio]ethanamin in wenig absoluten Ethanol gelöst versetzt und 2 Stunden unter Rückfluss erhitzt. Der Reaktionsansatz wird im Vakuum eingeengt und der Rückstand rotationschromatographisch gereinigt und man erhält die gereinigte Titelverbindung als zähes Öl; MS (+FAB-Methode): m/z (rel. Int. [%]) = 644 ($[M+H]^+$, 1), 121 (100). Zur weiteren Analytik wird ein Teil der Substanz in das Salz der Weinsäure überführt und aus Acetonitril/Isopropanol/Petrolether umkristallisiert; Smp.: 78°C (Acetonitril/Isopropanol/Petrolether).

Beispiel 100: N-Cyano-N′-[2-[[5-[(dimethylamino)methyl]furfuryl]thio]ethyl]-N″-[3-[N-[2-[N-(4-methoxybenzyl)-N-(2-pyridyl)amino]ethyl]-N-methylamino]propyl]guanidin

Die Herstellung erfolgt analog Beispiel 1 ausgehend von 0,55 g (1,6 mmol) N-[2-[N-(4-Methoxybenzyl)-N-(2-pyridyl)amino]ethyl]-N-methyl-1,3-propandiamin und der äquimolaren Menge N-Cyano-N′-[2-[[5-[(dimethylamino)methyl]furfuryl]thio]ethyl]-O-phenyl-isoharnstoff. Nach chromatographischer Aufarbeitung analog Beispiel 1 erhält man die gereinigte Titelverbindung als zähes Öl; MS (+FAB-Methode): m/z (rel. Int. [%]) = 593 ($[M+H]^+$, 4), 121 (100); IR (KBr): 2163 $cm^{-1}$ (C≡N). Zur weiteren Analytik wird ein Teil der Substanz in das Salz der Weinsäure überführt und aus Isopropanol/Ether umkristallisiert; Smp.: 74°C (Isopropanol/Ether).

Die Ausgangsverbindung wird wie folgt hergestellt:

Zu einer Suspension von ca. 0,1 mol N-Cyanodiphenylimidocarbonat in 100 ml Diethylether wird unter Rühren die äquimolare Menge 2-[[5-[(Dimethylamino)methyl]furfuryl]thio]ethanamin [J. Bradshaw et al., Br. J. Pharmacol. 66, 464P (1979)] zugegeben. Nach ca. 15 min Rühren kommt es zur massiven Fällung des Produktes N-Cyano-N′-[2-[[5-[(dimethylamino)methyl]furfuryl]thio]ethyl]-O-phenylisoharnstoff, oder es kristallisiert nach Einengen der Lösung. Man saugt ab, wäscht mit Diethylether und trocknet.

Beispiel 101: N-[2-[[5-[(Dimethylamino)methyl]furfuryl]thio]ethyl]-N′-[2-[N-[2-[N-(4-fluorbenzyl)-N-(2-pyridyl)amino]ethyl]-N-methylamino]ethyl]harnstoff

Die Herstellung erfolgt analog Beispiel 63 ausgehend von 0,56 g (1,8 mmol) N-[2-[N-(4-Fluorbenzyl)-N-(2-pyridyl)amino]ethyl]-N-methyl-1,2-ethandiamin und der äquimolaren Mengen 1,1′-Carbonyldiimidazol und 2-[[5-[(Dimethylamino)methyl]furfuryl]thio]ethylamin. Nach chromatographischer Aufarbeitung analog Beispiel 1 erhält man die gereinigte Titelverbindung als zähes Öl; MS (+FAB-Methode): m/z (rel. Int. [%]) = 543 ($[M+H]^+$, 14), 109 (100); IR (KBr): 1641 $cm^{-1}$ (C=O)
Zur weiteren Analytik wird ein Teil der Substanz in das Salz der O,O′-Ditoluoylweinsäure überführt und aus Isopropanol/Ether umkristallisiert; Smp.: 90°C (Isopropanol/Ether).

Beispiel 102: N-[2-[[5-[(Dimethylamino)methyl]furfuryl]thio]ethyl]-N′-[3-[N-[2-[N-(4-fluorbenzyl)-N-(2-pyridyl)amino]ethyl]-N-methylamino]propyl]harnstoff

Die Herstellung erfolgt analog Beispiel 63 ausgehend von 0,57 g (1,8 mmol) N-[2-[N-(4-Fluorbenzyl)-N-(2-pyridyl)amino]ethyl]-N-methyl-1,3-propandiamin und der äquimolaren Mengen 1,1′-Carbonyldiimidazol und 2-[[5-[(Dimethylamino)methyl]furfuryl]thio]ethylamin. Nach chromatographischer Aufarbeitung analog Beispiel 1 erhält man die gereinigte Titelverbindung als zähes Öl; MS (+FAB-Methode): m/z (rel. Int. [%]) = 557 ($[M+H]^+$, 1), 109 (100); IR (KBr): 1600 $cm^{-1}$ (C=O). Zur weiteren Analytik wird ein Teil der Substanz in das Salz der O,O′-Ditoluoylweinsäure in einem Lösungsmittelgemisch Ethanol/Ether überführt; Smp.: 102°C (Ethanol/Ether).

Beispiel 103: N-[2-[[5-[(Dimethylamino)methyl]furfuryl]thio]ethyl]-N′-[4-[N-[2-[N-(4-fluorbenzyl)-N-(2-pyridyl)amino]ethyl]-N-methylamino]butyl]harnstoff

Die Herstellung erfolgt analog Beispiel 63 ausgehend von 0,50 g (1,5 mmol) N-[2-[N-(4-Fluorbenzyl)-N-(2-pyridyl)amino]ethyl]-N-methyl-1,4-butandiamin und der äquimolaren Mengen 1,1′-Carbonyldiimidazol und 2-[[5-[(Dimethylamino)methyl]furfuryl]thio]ethylamin. Nach chromatographischer Aufarbeitung analog Beispiel 1 erhält man die gereinigte Titelverbindung als zähes Öl; MS (+FAB-Methode): m/z (rel. Int. [%]) = 571 ($[M+H]^+$, 17), 109 (100); IR (KBr): 1640 $cm^{-1}$ (C=O)
Zur weiteren Analytik wird ein Teil der Substanz in das Salz der Weinsäure in einem Lösungsmittelgemisch Ethanol/Ether überführt; Smp.: 74°C (Ethanol/Ether).

Beispiel 104: N-[2-[[5-[(Dimethylamino)methyl]furfuryl]thio]ethyl]-N′-[3-[N-[2-[N-(4-fluorbenzyl)-N-(2-pyridyl)amino]ethyl]-N-methylamino]propyl]-2-nitro-1,1-ethendiamin

Die Herstellung erfolgt analog Beispiel 22 ausgehend von 0,54 g (1,7 mmol) N-[2-[N-(4-Fluorbenzyl)-N-(2-pyridyl)amino]ethyl]-N-methyl-1,3-propandiamin und der äquimolaren Menge 1-[2-[[5-[(Dimethylamino) methyl]furfuryl]thio]ethyl]amino-1-methylthio-2-nitroethen. Nach chromatographischer Aufarbeitung analog Beispiel 22 erhält man die gereinigte Titelverbindung als zähes Öl; MS (+FAB-Methode): m/z (rel. Int. [%]) = 600 ($[M+H]^+$, 12), 109 (100). Zur weiteren Analytik wird ein Teil der Substanz in das Salz der Weinsäure in einem Lösungsmittelgemisch Ethanol/Ether überführt; Smp.: 63°C (Ethanol/Ether).

Beispiel 105: N-[2-[[(2-Guanidino-4-thiazolyl)methyl]thio]ethyl]-N′-[6-[N-methyl-N-[2-[1-(2-pyridyl)ethyl]inden-2-yl]ethylamino]hexyl]-2-nitro-1,1-ethendiamin

Die Herstellung erfolgt analog Beispiel 22 ausgehend von 0,60 g (2,6 mmol) 2-[[(2-Guanidino-4-thiazolyl)methyl]thio]ethanamin und der äquimolaren Menge 1-[[6-[N-Methyl-N-[[2-[1-(2-pyridyl)ethyl]inden-2-yl]ethyl]amino]hexyl]amino-1-methylthio-2-nitro-ethen. Nach chromatographischer Aufarbeitung analog Beispiel 22 erhält man die gereinigte Titelverbindung als Trockenschaum; MS (+FAB-Methode): m/z (rel. Int. [%]) = 678 ($[M+H]^+$, 11), 93 (100).

Die Ausgangsverbindung wird wie folgt hergestellt:

4,0 g (9,8 mmol) Dimethindenmaleat werden in Wasser gelöst. Die Lösung wird durch 2N NaOH-Lösung alkalisiert und viermal mit n-Hexan ausgeschüttelt. Die vereinigten organischen Phasen werden mit $Na_2SO_4$ getrocknet und das Lösungsmittel im Vakuum abdestilliert. Die freie Base wird in 10 ml absolutem 1,2-Dichlorethan gelöst und zu dieser Lösung eine Spatelspitze $Na_2CO_3$ (wasserfrei) zugegeben. Die Mischung wird auf 0°C gekühlt und bei dieser Temperatur 2,79 g (19,6 mmol) $\alpha$-Chlorethylchloroformat zugetropft. Danach wird der Ansatz 12 Stunden unter Rückfluss erhitzt. Nach dem Abkühlen wird überschüssiges $Na_2CO_3$ abfiltriert und das Lösungsmittel im Vakuum abdestilliert. Der ölige Rückstand wird über Nacht in Methanol unter Rück-

fluss erhitzt, wobei $CO_2$ frei wird. Das Methanol wird schliesslich im Vakuum abdestilliert und man erhält 2,7 g N-Methyl-2-[3-[1-(2-pyridyl)ethyl]inden-2-yl]ethanamin [S. Radler, Dissertation, Westfälische Wilhelms-Universität Münster (1989)] in Form des Hydrochlorides als farbloses Öl, welches mittels 6-Bromhexannitril und anschliessender $LiAlH_4$-Behandlung analog des in Beispiel 96 angegebenen Verfahrens zu N-Methyl-N-[2-[3-[1-(2-pyridyl)ethyl]inden-2-yl]ethyl]-1,6-hexandiamin und (durch Reaktion mit 1,1-Dimethylthio-2-nitroethen) weiter zum 1-[[6-[N-Methyl-N-[[2-[1-(2-pyridyl)ethyl]inden-2-yl]ethyl]amino]hexyl]amino-1-methylthio-2-nitroethen umgesetzt wird.

Beispiel 106: N-[7-[N-[3-(4-Chlorphenyl)-3-(2-pyridyl)propyl]-N-methylamino]heptyl]-N'-[3-[3-(piperidinomethyl)phenoxy]propyl]-2-nitro-1,1-ethendiamin

Eine Mischung aus 0,73 g (1,9 mmol) N-[3-(4-Chlorphenyl)-3-(2-pyridyl)propyl]-N-methyl-1,7-heptandiamin und einer äquimolaren Menge 1-Methylthio-1-[3-[3-(piperidinomethyl)phenoxy]propyl]amino-2-nitroethen werden in 20 ml Acetonitril 12 Stunden unter Rückfluss erhitzt. Danach wird der Ansatz vom Lösungsmittel befreit und die Titelverbindung mittels präparativer Dickschichtchromatographie (Eluens: Ethylacetat/Methanol 9+1, Ammoniakatmosphäre) als zähes Öl isoliert; MS (+FAB-Methode): m/z ( rel. Int.[%]) = 691 ($[M+H]^+$ 12), 230 (100).

Beispiel 107: Eine Salbe enthaltend 0,05 Gewichtsprozent Wirkstoff, z.B. N-[3-[N-[3-(4-Chlorphenyl)-3-(2-pyridyl)propyl]-N-methylamino]propyl]-N'-[3-[3-(piperidinomethyl)phenoxy]propyl]harnstoff, wird wie folgt hergestellt:

| Zusammensetzung | Gewichtsprozent |
|---|---|
| Wirkstoff | 0,05 |
| Vaseline | 45,00 |
| Paraffinöl | 19,60 |
| Cetylalkohol | 5,00 |
| Bienenwachs | 5,00 |
| Sorbitan-sesquioleat | 5,00 |
| p-Hydroxybenzoesäureester | 0,20 |
| Wasser, entmineralisiert | 20,15 |

Die Fettstoffe und Emulgatoren werden zusammengeschmolzen. Das Konservierungsmittel wird in Wasser gelöst und die Lösung in die Fettschmelze bei erhöhter Temperatur einemulgiert. Nach dem Erkalten wird eine Suspension des Wirkstoffs in einem Teil der Fettschmelze in die Emulsion eingearbeitet.

Beispiel 108: Tabletten enthaltend je 50 mg Wirkstoff, z.B. N-[7-[N-[3-(4-Chlorphenyl)-3-(2-pyridyl)propyl]-N-methylamino]heptyl]-N'-[2-[[(2-guanidino-4-thiazolyl)methyl]thio]ethyl]harnstoff, werden wie folgt hergestellt:

Zusammensetzung (10000 Tabletten)

| | |
|---|---|
| Wirkstoff | 500,0 g |
| Lactose | 500,0 g |
| Kartoffelstärke | 352,0 g |
| Gelatine | 8,0 g |
| Talk | 60,0 g |
| Magnesiumstearat | 10,0 g |
| Siliciumdioxid (hochdispers) | 20,0 g |
| Ethanol | q.s. |

Der Wirkstoff wird mit der Lactose und 292 g der Kartoffelstärke vermischt und die Mischung mit einer ethanolischen Lösung der Gelatine befeuchtet und durch ein Sieb granuliert. Nach dem Trocknen mischt man den Rest der Kartoffelstärke, das Magnesiumstearat, den Talk und das Siliciumdioxid zu und presst das Gemisch zu Tabletten von je 145 mg Gewicht und 50 mg Wirkstoffgehalt, die gewünschtenfalls mit Teilkerben zur feineren Anpassung der Dosierung versehen sein können.

## Patentansprüche

1. Arylalkylaminderivate der allgemeinen Formel I,

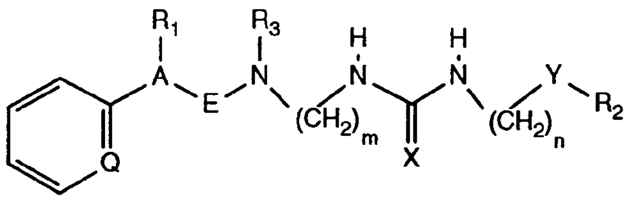

(I)

in der $R_1$ für eine substituierte oder unsubstituierte Aryl-, Heteroaryl-, Aryl-$C_1$-$C_3$-alkyl-oder Heteroaryl-$C_1$-$C_3$-alkyl-gruppe, ein Wasserstoffatom oder eine $C_1$-$C_3$-Alkylgruppe stent, A ein Stickstoffatom oder eine CH-Gruppe bedeutet, E für die Gruppierung $-(CH_2)_p-$, $-O-(CH_2)_p-$, $-S-(CH_2)_p-$ oder

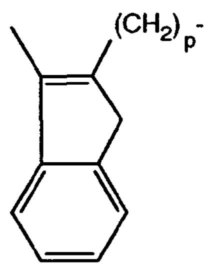

steht, wobei p den Wert 2, 3 oder 4 haben kann, Q für ein Stickstoffatom oder eine CH-Gruppe steht, $R_2$ eine gegebenenfalls basisch substituierte Aryl-, Heteroaryl-, Aryl-$C_1$-$C_3$-alkyl- oder Heteroaryl-$C_1$-$C_3$-alkyl-gruppe, $R_3$ ein Wasserstoffatom oder eine $C_1$-$C_3$-Alkylgruppe, X ein Sauerstoffatom, ein Schwefelatom, die Gruppierung N-CN oder $CH-NO_2$ bedeutet, m den Wert 2, 3, 4, 5, 6, 7 oder 8 und n den Wert 1, 2, 3 oder 4 haben kann und Y ein Schwefelatom, ein Sauerstoffatom oder eine Methylengruppe bedeutet, sowie deren stereoisomere Formen, Hydrate und physiologisch annehmbare Salze.

2. Arylalkylaminderivate der Formel I nach Anspruch 1, worin $R_1$ für Phenyl, Furyl, Thienyl, Phenyl-$C_1$-$C_3$-alkyl oder (Furyl, Thienyl oder Pyridyl)-$C_1$-$C_3$-alkyl steht, wobei der Phenylring bzw. Heteroarylring jeweils unsubstituiert oder einfach oder zweifach durch Halogen, $C_1$-$C_3$-Alkyl und/oder $C_1$-$C_3$-Alkoxy substituiert ist, oder für Wasserstoff oder $C_1$-$C_3$-Alkyl steht, A ein Stickstoffatom oder eine CH-Gruppe bedeutet, E für die Gruppierung $-(CH_2)_p-$, $-O-(CH_2)_p-$, $-S-(CH_2)_p-$ oder

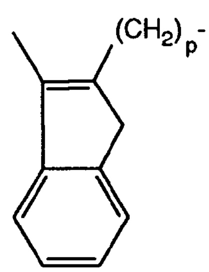

steht, wobei p den Wert 2, 3 oder 4 hat, Q für ein Stickstoffatom oder eine CH-Gruppe steht, $R_2$ Piperidino-$C_1$-$C_3$-alkyl-phenyl, Guanidino-thiazolyl-$C_1$-$C_3$-alkyl, $C_1$-$C_3$-Alkyl-imidazolyl-$C_1$-$C_3$-alkyl oder (N,N-Di-$C_1$-$C_3$-alkylamino-$C_1$-$C_3$-alkyl)-furanyl-$C_1$-$C_3$-alkyl bedeutet, $R_3$ für Wasserstoff oder $C_1$-$C_3$-Alkyl steht, X ein Sauerstoffatom, ein Schwefelatom, die Gruppierung N-CN oder $CH-NO_2$ bedeutet, m den Wert 2, 3, 4,

5, 6, 7 oder 8 und n den Wert 1, 2, 3 oder 4 hat, und Y ein Schwefelatom, ein Sauerstoffatom oder eine Methylengruppe bedeutet, sowie deren stereoisomere Formen, Hydrate und physiologisch anenhmbare Salze.

3. Arylalkylaminderivate nach Anspruch 1, dadurch gekennzeichnet, dass $R_1$ für einen unsubstituierten oder einen einfach oder zweifach mit Halogenatomen, $C_1$-$C_3$-Alkyl- oder $C_1$-$C_3$-Alkoxygruppen substituierten Phenyl-, Furyl- oder Thienylring steht, A eine CH-Gruppe oder ein Stickstoffatom bedeutet, Q für ein Stickstoffatom oder eine CH-Gruppe steht, und E, $R_2$, $R_3$, X, Y, m, n und p die in Anspruch 1 genannte Bedeutung besitzen.

4. Arylalkylaminderivate nach Anspruch 1, dadurch gekennzeichnet, dass $R_1$ für eine unsubstituierte oder eine einfach oder zweifach mit Halogenatomen, $C_1$-$C_3$-Alkyl- oder $C_1$-$C_3$-Alkoxygruppen substituierte Benzyl-, Furylmethyl-, Thienylmethyl- oder Pyridylmethylgruppe steht, A ein Stickstoffatom bedeutet, Q für ein Stickstoffatom steht, und E, $R_2$, $R_3$, X, Y, m, n und p die in Anspruch 1 genannte Bedeutung besitzen.

5. Arylalkylaminderivate der Formel I nach Anspruch 1, worin $R_1$ für Phenyl oder Phenyl-$C_1$-$C_3$-alkyl, worin der Phenylring jeweils unsubstituiert durch Halogen, $C_1$-$C_3$-Alkyl oder $C_1$-$C_3$-Alkoxy substituiert ist, oder für Wasserstoff oder $C_1$-$C_3$-Alkyl steht, A ein Stickstoffatom oder eine CH-Gruppe bedeutet, E für die Gruppierung $-(CH_2)_p-$, $-O-(CH_2)_p-$, $-S-(CH_2)_p-$ oder

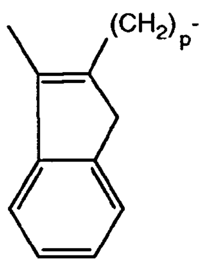

steht, wobei p den Wert 2 oder 3 hat, Q für ein Stickstoffatom oder eine CH-Gruppe steht, $R_2$ Piperidino-$C_1$-$C_3$-alkyl-phenyl, Guanidino-thiazolyl-$C_1$-$C_3$-alkyl, $C_1$-$C_3$-Alkyl-imidazolyl-$C_1$-$C_3$-alkyl oder (N,N-Di-$C_1$-$C_3$-alkylamino-$C_1$-$C_3$-alkyl)-furanyl-$C_1$-$C_3$-alkyl bedeutet, $R_3$ für Wasserstoff oder $C_1$-$C_3$-Alkyl steht, X ein Sauerstoffatom, ein Schwefelatom, die Gruppierung N-CN oder $CH-NO_2$ bedeutet, m den Wert 2, 3, 4, 5, 6, 7 oder 8 und n den Wert 1,2, 3 oder 4 haben kann, und Y ein Schwefelatom oder ein Sauerstoffatom bedeutet, sowie deren stereoisomere Formen, Hydrate und physiologisch annehmbare Salze.

6. Arylalkylaminderivate der Formel I nach Anspruch 1, worin $R_1$ für Phenyl, das unsubstituiert oder durch Halogen oder $C_1$-$C_3$-Alkyl substituiert ist, Phenyl-$C_1$-$C_3$-alkyl, das im Phenylring durch Halogen oder $C_1$-$C_3$-Alkoxy substituiert ist, oder für $C_1$-$C_3$-Alkyl steht, A ein Stickstoffatom oder eine CH-Gruppe bedeutet, E für die Gruppierung $-(CH_2)_p-$, $-O-(CH_2)_p-$, $-S-(CH_2)_p-$ oder

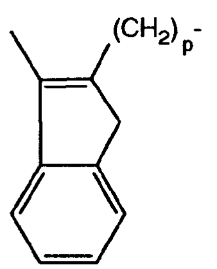

steht, wobei p den Wert 2 hat, Q für ein Stickstoffatom oder eine CH-Gruppe steht, $R_2$ 3-Piperidinomethyl-phenyl, 2-Guanidino-thiazol-4-ylmethyl, 5-Methyl-imidazol-4-ylmethyl oder 5-(N,N-Dimethylaminomethyl)-furan-2-ylmethyl bedeutet, $R_3$ für $C_1$-$C_3$-Alkyl steht, X ein Sauerstoffatom, ein Schwefelatom, die Gruppierung N-CN oder $CH-NO_2$ bedeutet, m den Wert 2, 3, 4, 5, 6, 7 oder 8 und n den Wert 2, 3 oder 4 haben kann, und Y ein Schwefelatom oder ein Sauerstoffatom bedeutet, sowie deren stereoisomere Formen, Hydrate und physiologisch annehmbare Salze.

**7.** Arylalkylaminderivate nach Anspruch 1, dadurch gekennzeichnet, dass $R_1$ für ein Wasserstoffatom oder eine $C_1$-$C_3$-Alkylgruppe, A für eine CH-Gruppe und Q für ein Stickstoffatom steht, E die Gruppierung

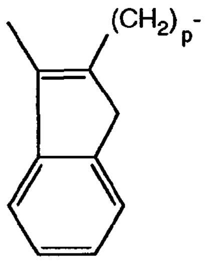

bedeutet und $R_2$, $R_3$, X, Y, m, n und p die in Anspruch 1 genannte Bedeutung besitzen.

**8.** Arylalkylaminderivate nach Anspruch 1, dadurch gekennzeichnet, dass $R_2$ für die Gruppierung 5-Methyl-imidazol-4-ylmethyl steht und $R_1$, $R_3$, A, E, Q, X, Y, m, n und p die in Anspruch 1 genannte Bedeutung besitzen.

**9.** Arylalkylaminderivate nach Anspruch 1, dadurch gekennzeichnet, dass $R_2$ für die Gruppierung 2-Guanidino-thiazol-4-ylmethyl steht und $R_1$, $R_3$, A, E, Q, X, Y, m, n und p die in Anspruch 1 genannte Bedeutung besitzen.

**10.** Arylalkylaminderivate nach Anspruch 1, dadurch gekennzeichnet, dass $R_2$ für die Gruppierung 5-(N,N-Dimethylaminomethyl)-furan-2-ylmethyl steht und $R_1$, $R_3$, A, E, Q, X, Y, m, n und p die in Anspruch 1 genannte Bedeutung besitzen.

**11.** Arylalkylaminderivate nach Anspruch 1, dadurch gekennzeichnet, dass $R_2$ für die Gruppierung 3-Piperidinomethyl-phenyl steht und $R_1$, $R_3$, A, E, Q, X, Y, m, n und p die in Anspruch 1 genannte Bedeutung besitzen.

**12.** N-[7-[N-[3-(4-Chlorphenyl)-3-(2-pyridyl)propyl]-N-methylamino]heptyl]-N'-[2-[[(2-guanidino-4-thiazolyl) methyl]thio]ethyl]harnstoff gemäss Anspruch 1 oder ein pharmazeutisch verwendbares Salz davon.

**13.** N-[2-[[2-Guanidino-4-thiazolyl)methyl]thio]ethyl]-N'-[6-[N-(4-methoxybenzyl)-N-(2-pyridyl)amino]ethyl]-N-methylamino]hexyl]-2-nitro-1,1-ethendiamin gemäss Anspruch 1 oder ein pharmazeutisch verwendbares Salz davon.

**14.** Pharmazeutisches Präparat enthaltend eine Verbindung gemäss einem der Ansprüche 1 bis 13 und mindestens ein pharmazeutisch verwendbares Trägermaterial.

**15.** Pharmazeutisches Präparat gemäss Anspruch 14 enthaltend N-[7-[N-[3-(4-Chlorphenyl)-3-(2-pyridyl)propyl]-N-methylamino]heptyl]-N'-[2-[[2-guanidino-4-thiazolyl)methyl]thio]ethyl]harnstoff oder ein pharmazeutisch verwendbares Salz davon.

**16.** Pharmazeutisches Präparat gemäss Anspruch 14 enthaltend N-[2-[[(2-Guanidino-4-thiazolyl)methyl]thio]ethyl]-N'-[6-[N-[2-[N-(4-methoxybenzyl)-N-(2-pyridyl)amino]ethyl]-N-methylamino]hexyl]-2-nitro-1,1-ethendiamin oder ein pharmazeutisch verwendbares Salz davon.

**17.** Eine Verbindung gemäss einem der Ansprüche 1 bis 13 zur Anwendung in einem Verfahren zur therapeutischen Behandlung des tierischen oder menschlichen Körpers.

**18.** Eine Verbindung gemäss einem der Ansprüche 1 bis 13 zur Verwendung bei der Behandlung von Krankheiten, die auf eine Hemmung der Histamin-$H_1$- und $H_2$-Rezeptoren ansprechen.

**19.** Verwendung einer Verbindung gemäss einem der Ansprüche 1 bis 13 zur Herstellung pharmazeutischer Präparate.

**20.** Verwendung einer Verbindung gemäss einem der Ansprüche 1 bis 13 zur Herstellung pharmazeutischer Präparate für die Behandlung von Krankheiten, die auf eine Hemmung der Histamin-$H_1$- und $H_2$-Rezeptoren ansprechen.

**21.** Verfahren zur Herstellung einer Verbindung der Formel I gemäss Anspruch 1, oder einem Stereoisomer, Hydrat oder einem physiologisch annehmbaren Salz davon, dadurch gekennzeichnet, dass man

a) zur Herstellung einer Verbindung der Formel I, in der X für ein Sauerstoff- oder ein Schwefelatom, die Gruppierung N-CN oder $CH-NO_2$ steht,

(a1) eine Verbindung der Formel II

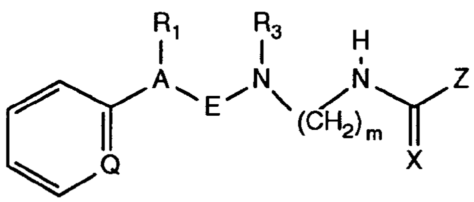

(II)

in der $R_1$, $R_3$, A, E, Q und m die in Anspruch 1 genannte Bedeutung besitzen und Z für eine Methylthio-, eine Mercapto- oder eine Phenoxygruppe steht, mit einer Verbindung der allgemeinen Formel III

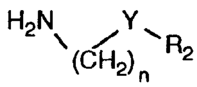

(III)

in der $R_2$, Y und n die in Anspruch 1 angegebene Bedeutung besitzen, umsetzt, oder

(a2)eine Verbindung der Formel IV,

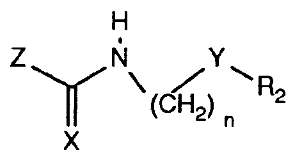

(IV)

in der $R_2$, Y und n die in Anspruch 1 angegebene Bedeutung besitzen und Z für eine Methylthio-, eine Mercapto- oder eine Phenoxygruppe steht, mit einer Verbindung der allgemeinen Formel V,

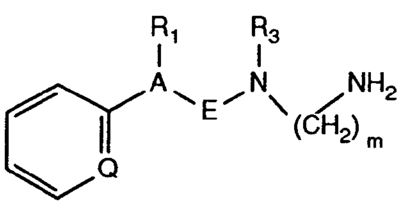

(V)

in der $R_1$, $R_3$, A, E, Q und m die in Anspruch 1 genannte Bedeutung besitzen, umsetzt, oder

b) zur Herstellung einer Verbindung der Formel I, in der X für ein Sauerstoff- oder ein Schwefelatom steht,

(b1) eine Verbindung der Formel VI,

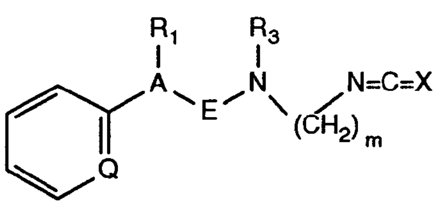

(VI)

in der $R_1$, $R_3$, A, E, Q und m die in Anspruch 1 genannte Bedeutung besitzen, mit einer Verbindung

der Formel III, in der $R_2$, Y und n die in Anspruch 1 angegebene Bedeutung besitzen, umsetzt, oder (b2) eine Verbindung der Formel VII,

$$X = C = N - (CH_2)_n - Y - R_2 \qquad (VII)$$

in der $R_2$, Y und n die in Anspruch 1 angegebene Bedeutung besitzen, mit einer Verbindung der Formel V

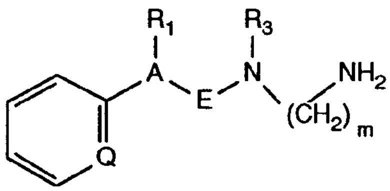

(V)

in der $R_1$, $R_3$, A, E, Q und m die in Anspruch 1 genannte Bedeutung Besitzen, umsetzt,

und, wenn erwünscht, eine erhaltene Verbindung der Formel I in eine andere Verbindung der Formel I umwandelt, und/oder, wenn erwünscht, ein erhaltenes Salz in die freie Verbindung oder in ein anderes Salz umwandelt, und/oder, wenn erwünscht, eine erhaltene freie Verbindung der Formel I mit salzbildenden Eigenschaften in ein Salz umwandelt.

Europäisches Patentamt

# EUROPÄISCHER RECHERCHENBERICHT

Nummer der Anmeldung
EP 92 81 0536

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| A | EP-A-0 262 448 (HEUMANN PHARMA GMBH & CO)<br>* Ansprüche 1,17,18; Beispiele *<br>--- | 1-21 | C07D213/38<br>C07D213/74<br>C07D277/42 |
| A | EP-A-0 214 823 (FUJIREBIO KABUSHIKI KAISHA)<br>* Zusammenfassung; Ansprüche 1,14-17; Beispiel 109 *<br>--- | 1-21 | C07D401/12<br>C07D405/12<br>C07D417/12<br>A61K31/425<br>A61K31/44 |
| A | EP-A-0 101 379 (SANOFI SA)<br>* das ganze Dokument *<br>----- | 1-21 | //(C07D417/12, 277:00, 239:00)<br>(C07D405/12, 307:00, 213:00)<br>(C07D401/12, 233:00, 213:00) |

RECHERCHIERTE SACHGEBIETE (Int. Cl.5)

C07D
A61K

Der vorliegende Recherchenbericht wurde für alle Patentansprüche erstellt

| Recherchenort | Abschlußdatum der Recherche | Prüfer |
|---|---|---|
| DEN HAAG | 20 OKTOBER 1992 | BOSMA P. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)